# EUROPEAN PATENT SPECIFICATION

(11) **EP 1 601 764 B1**
(45) Date of publication and mention of the grant of the patent: **28.04.2010**
(21) Application number: 04718969.1
(22) Date of filing: 10.03.2004
(51) Int. Cl.: C12N 9/50

(54) **A CLASS OF METACASPASES**
EINE METACASPASEN-KLASSE
CLASSE DE METACASPASES

(30) Priority: 11.03.2003 EP 03075723
(43) Date of publication of application: 07.12.2005
(73) Proprietor: VIB, vzw, 9052 Zwijnaarde (BE); Universiteit Gent, 9000 Gent (BE)
(72) Inventor: INZE, Dirk, Gustaaf, B-9310 Moorsel - Aalst (BE); VAN BREUSEGEM, Frank, B-9660 Brakel (BE); VERCAMMEN, Dominique, B-9052 Zwijnaarde (BE); VAN DE COTTE, Brigitte, B-9770 Kruishoutem (BE)
(86) International application number: PCT/EP2004/050285
(87) International publication number: WO 2004/081168

(56) References cited:
- SZALLIES A ET AL: "A metacaspase of Trypanosoma brucei causes loss of respiration competence and clonal death in the yeast Saccharomyces cerevisiae" FEBS LETTERS, ELSEVIER SCIENCE PUBLISHERS, AMSTERDAM, NL, vol. 517, no. 1-3, 24 April 2002 (2002-04-24), pages 144-150, XP004350277 ISSN: 0014-5793
- WOLTERING ERNST J ET AL: "Do plant caspases exist?" PLANT PHYSIOLOGY (ROCKVILLE), vol. 130, no. 4, December 2002 (2002-12), pages 1764-1769, XP002289433 ISSN: 0032-0889
- DATABASE UNIPROT [Online] Putative latex-abundant protein 6 March 2001 (2001-03-06), XP002289434 Database accession no. Q9FYE1
- EICHINGER ANDREAS ET AL: "Crystal structure of gingipain R: An Arg-specific bacterial cysteine proteinase with a caspase-like fold" EMBO JOURNAL, OXFORD UNIVERSITY PRESS, SURREY, GB, vol. 18, no. 20, 15 October 1999 (1999-10-15), pages 5453-5462, XP002152750 ISSN: 0261-4189
- UREN A G ET AL: "Identification of paracaspases and metacaspases: two ancient families of caspase-like proteins, one of which plays a key role in MALT lymphoma" MOLECULAR CELL, CELL PRESS, CAMBRIDGE, MA, US, vol. 6, no. 4, October 2000 (2000-10), pages 961-967, XP002207100 ISSN: 1097-2765 cited in the application
- SOLOMON ET AL.: "The Involvement of Cysteine Proteases and Protease Inhibitor Genes in the Regulation of Programmed Cell Death in Plants" THE PLANT CELL, vol. 11, 1999, pages 431-443,

### SEQUENCE LISTING

<110> VIB vzw
<120> A NOVEL CLASS OF METACASPASES
<130> FvB/Met/v147
<150> EP 03075723.1
   <151> 2003-03-11
<160> 45
<170> PatentIn version 3.1
<210> 1
   <211> 343
   <212> PRT
   <213> Arabidopsis thaliana
<220>
   <221> MISC_FEATURE
   <223> Atmc9
<400> 1
   His His His His His Leu Glu Ser Thr Ser Leu Tyr Lys Lys Ala Gly
   1               5                   10                  15
   Ser Thr Met Asp Gln Gln Gly Met Val Lys Lys Arg Leu Ala Val Leu
               20                  25                  30
   Val Gly Cys Asn Tyr Pro Asn Thr Arg Asn Glu Leu His Gly Cys Ile
           35                  40                  45
   Asn Asp Val Leu Ala Met Lys Glu Thr Ile Leu Ser Arg Phe Gly Phe
       50                  55                  60
   Lys Gln Asp Asp Ile Glu Val Leu Thr Asp Glu Pro Glu Ser Lys Val
   65                  70                  75                  80
   Lys Pro Thr Gly Ala Asn Ile Lys Ala Ala Leu Arg Arg Met Val Asp
                   85                  90                  95
   Lys Ala Gln Ala Gly Ser Gly Asp Ile Leu Phe Phe His Tyr Ser Gly
               100                 105                 110
   His Gly Thr Arg Ile Pro Ser Val Lys Ser Ala His Pro Phe Lys Gln
           115                 120                 125
   Asp Glu Ala Ile Val Pro Cys Asp Phe Asn Leu Ile Thr Asp Val Asp
       130                 135                 140
   Phe Arg Glu Leu Val Asn Gln Leu Pro Lys Gly Thr Ser Phe Thr Met
   145                 150                 155                 160
   Ile Ser Asp Ser Cys His Ser Gly Gly Leu Ile Asp Lys Glu Lys Glu
                   165                 170                 175
   Gln Ile Gly Pro Ser Ser Val Ser Ser Asn Ile Ser Pro Ala Ile Glu
               180                 185                 190
   Thr Thr Asn Lys Thr Ile Thr Ser Arg Ala Leu Pro Phe Lys Ala Val
           195                 200                 205
   Leu Asp His Leu Ser Ser Leu Thr Gly Ile Thr Thr Ser Asp Ile Gly
       210                 215                 220
   Thr His Leu Leu Glu Leu Phe Gly Arg Asp Ala Gly Leu Lys Phe Arg
   225                 230                 235                 240
   Leu Pro Ala Met Asp Leu Met Asp Leu Leu Glu Thr Met Thr Ala Arg
                   245                 250                 255
   Glu Lys His Val Asp Ser Gly Ile Leu Met Ser Gly Cys Gln Ala Asp
               260                 265                 270
   Glu Thr Ser Ala Asp Val Gly Val Gly Asn Gly Lys Ala Tyr Gly Ala
           275                 280                 285
   Phe Ser Asn Ala Ile Gln Arg Val Leu Asn Glu Asn Glu Gly Ala Met
       290                 295                 300
   Lys Asn Lys Gln Leu Val Met Met Ala Arg Asp Val Leu Glu Arg Leu
   305                 310                 315                 320
   Gly Phe His Gln His Pro Cys Leu Tyr Cys Ser Asp Gln Asn Ala Asp
                   325                 330                 335
   Ala Thr Phe Leu Ser Gln Pro
               340
<210> 2
   <211> 142
   <212> PRT
   <213> Arabidopsis thaliana
<220>
   <221> MIST_FEATURE
   <223> part of Atmc9
<400> 2
   Ala Leu Pro Phe Lys Ala Val Leu Asp His Leu Ser Ser Leu Thr Gly
   1               5                   10                  15
   Ile Thr Thr Ser Asp Ile Gly Thr His Leu Leu Glu Leu Phe Gly Arg
               20                  25                  30
   Asp Ala Gly Leu Lys Phe Arg Leu Pro Ala Met Asp Leu Met Asp Leu
           35                  40                  45
   Leu Glu Thr Met Thr Ala Arg Glu Lys His Val Asp Ser Gly Ile Leu
       50                  55                  60
   Met Ser Gly Cys Gln Ala Asp Glu Thr Ser Ala Asp Val Gly Val Gly
   65                  70                  75                  80
   Asn Gly Lys Ala Tyr Gly Ala Phe Ser Asn Ala Ile Gln Arg Val Leu
                   85                  90                  95
   Asn Glu Asn Glu Gly Ala Met Lys Asn Lys Gln Leu Val Met Met Ala
               100                 105                 110
   Arg Asp Val Leu Glu Arg Leu Gly Phe His Gln His Pro Cys Leu Tyr
           115                 120                 125
   Cys Ser Asp Gln Asn Ala Asp Ala Thr Phe Leu Ser Gln Pro
       130                 135                 140
<210> 3
   <211> 418
   <212> PRT
   <213> Arabidopsis thaliana
<220>
   <221> MIST_FEATURE
   <223> Atmc4
<400> 3
   Met Thr Lys Lys Ala Val Leu Ile Gly Ile Asn Tyr Pro Gly Thr Lys
   1               5                   10                  15
   Ala Glu Leu Arg Gly Cys Val Asn Asp Val Arg Arg Met Tyr Lys Cys
               20                  25                  30
   Leu Val Glu Arg Tyr Gly Phe Ser Glu Glu Asn Ile Thr Val Leu Ile
           35                  40                  45
   Asp Thr Asp Glu Ser Ser Thr Gln Pro Thr Gly Lys Asn Ile Arg Arg
       50                  55                  60
   Ala Leu Ala Asp Leu Val Glu Ser Ala Asp Ser Gly Asp Val Leu Val
   65                  70                  75                  80
   Val His Tyr Ser Gly His Gly Thr Arg Leu Pro Ala Glu Thr Gly Glu
                   85                  90                  95
   Asp Asp Asp Thr Gly Phe Asp Glu Cys Ile Val Pro Cys Asp Met Asn
               100                 105                 110
   Leu Ile Thr Asp Asp Asp Phe Arg Asp Leu Val Asp Lys Val Pro Pro
           115                 120                 125
   Gly Cys Arg Met Thr Ile Ile Ser Asp Ser Cys His Ser Gly Gly Leu
       130                 135                 140
   Ile Asp Glu Ala Lys Glu Gln Ile Gly Glu Ser Thr Lys Lys Glu Ala
   145                 150                 155                 160
   Glu Asp Glu Asp Glu Ser Glu Glu Ser Ser Ser Arg Phe Gly Phe Arg
                   165                 170                 175
   Lys Phe Leu Arg Ser Lys Val Glu Gly Ala Ile Glu Ser Arg Gly Phe
               180                 185                 190
   His Ile Gly Gly Asn Lys Lys Asp Glu Asp Val Ala Glu Glu Ile Glu
           195                 200                 205
   Thr Lys Glu Ile Glu Leu Glu Asp Gly Glu Thr Ile His Ala Lys Asp
       210                 215                 220
   Lys Ser Leu Pro Leu Gln Thr Leu Ile Asp Ile Leu Lys Gln Gln Thr
   225                 230                 235                 240
   Gly Asn Asp Asn Ile Glu Val Gly Lys Ile Arg Pro Ser Leu Phe Asp
                   245                 250                 255
   Ala Phe Gly Asp Asp Ser Ser Pro Lys Val Lys Lys Phe Met Lys Val
               260                 265                 270
   Ile Leu Gly Lys Leu Gln Ala Gly Asn Gly Glu Glu Gly Gly Leu Met
           275                 280                 285
   Gly Met Leu Gly Lys Leu Ala Ser Gly Phe Leu Glu Gly Lys Leu Asn
       290                 295                 300
   Asp Glu Asp Tyr Val Lys Pro Ala Met Gln Thr His Val Gly Ser Lys
   305                 310                 315                 320
   Glu Glu Val Tyr Ala Gly Gly Ser Arg Gly Ser Val Pro Leu Pro Asp
                   325                 330                 335
   Ser Gly Ile Leu Ile Ser Gly Cys Gln Thr Asp Gln Thr Ser Ala Asp
               340                 345                 350
   Ala Thr Pro Ala Gly Lys Pro Thr Glu Ala Tyr Gly Ala Met Ser Asn
           355                 360                 365
   Ser Ile Gln Thr Ile Leu Glu Glu Thr Asp Gly Glu Ile Ser Asn Arg
       370                 375                 380
   Glu Met Val Thr Arg Ala Arg Lys Ala Leu Lys Lys Gln Gly Phe Thr
   385                 390                 395                 400
   Gln Gln Pro Gly Leu Tyr Cys His Asp Gly Tyr Ala Asn Ala Pro Phe
                   405                 410                 415
   Ile Cys
<210> 4
   <211> 410
   <212> PRT
   <213> Arabidopsis thaliana
<220>
   <221> MIST_FEATURE
   <223> Atmc5
<400> 4
   Met Ala Lys Lys Ala Val Leu Ile Gly Ile Asn Tyr Pro Gly Thr Lys
   1               5                   10                  15
   Ala Glu Leu Arg Gly Cys Val Asn Asp Val Arg Arg Val His Lys Ser
               20                  25                  30
   Leu Val Asp Arg Phe Gly Phe Ser Glu Arg Asn Ile Thr Glu Leu Ile
           35                  40                  45
   Asp Thr Asp Glu Ser Ser Thr Lys Pro Thr Gly Lys Asn Ile Arg Arg
       50                  55                  60
   Ala Leu Leu Asn Leu Val Glu Ser Ala Lys Pro Gly Asp Val Leu Val
   65                  70                  75                  80
   Val His Tyr Ser Gly His Gly Thr Arg Leu Pro Ala Glu Thr Gly Glu
                   85                  90                  95
   Asp Asp Asp Thr Gly Tyr Asp Glu Cys Ile Val Pro Cys Asp Met Asn
               100                 105                 110
   Leu Ile Thr Asp Asp Glu Phe Arg Asp Leu Val Glu Lys Val Pro Lys
           115                 120                 125
   Glu Ala His Ile Thr Ile Ile Ser Asp Ser Cys His Ser Gly Gly Leu
       130                 135                 140
   Ile Asp Glu Ala Lys Glu Gln Ile Gly Glu Ser Thr Lys Lys Lys Pro
   145                 150                 155                 160
   Lys Lys Glu Ser Gly Gly Ser Ser Gly Leu Gly Ile Lys Gly Phe Val
   165 170 175
   Arg Glu Ala Val Glu Glu Ala Leu Glu Ser Lys Gly Ile Ala Ile Pro
   180 185 190
   His His Lys Asp Glu Lys Asp Glu Asn Lys Thr Lys Glu Leu Lys Leu
   195 200 205
   Glu Asp Gly Ala Lys Val His Val Val Asn Lys Ser Leu Pro Leu Gln
   210 215 220
   Thr Leu Ile Asp Ile Leu Lys Gln Asn Thr Gly Asn Asn Asp Ile Glu
   225 230 235 240
   Val Gly Lys Ile Arg Pro Thr Leu Phe Asn Val Phe Gly Glu Asp Ala
   245 250 255
   Ser Pro Lys Val Lys Lys Phe Met Lys Val Ile Leu Thr Lys Leu Gln
   260 265 270
   Glu Gly Lys Thr Glu Gly Gly Ile Leu Gly Met Ile Gly Lys Leu Ala
   275 280 285
   Gln Glu Phe Leu Lys His Lys Leu Asn Asp Asp Glu Glu Tyr Val Lys
   290 295 300
   Pro Ala Met Lys Thr His Val Gly Asn Lys Gln Glu Val Tyr Ala Gly
   305 310 315 320
   Ala Ser Asn Gly Ser Leu Ala Asp Asn Gly Ile Leu Ile Ser Gly Cys
   325 330 335
   Gln Thr Asp Gln Thr Ser Ala Asp Ala Ser Pro Gln Gly His Pro Glu
   340 345 350
   Met Ala Tyr Gly Ala Phe Thr Asn Ala Val Gln Ile Ile Leu Glu Glu
   355 360 365
   Thr Lys Gly Met Ile Thr Tyr Lys Glu Leu Val Leu Lys Ala Arg Lys
   370 375 380
   Leu Leu Lys Lys Gln Gly Phe Ser Gln Arg Pro Gly Leu Tyr Cys Ser
   385 390 395 400
   Asp Ser Phe Val Asn Ala Pro Phe Ile Cys
   405 410
<210> 5
   <211> 368
   <212> PRT
   <213> Arabidopsis thaliana
<220>
   <221> MIST_FEATURE
   <223> Atmc6
<400> 5
   Met Ala Lys Lys Ala Leu Leu Ile Gly Ile Asn Tyr Val Gly Thr Lys
   1               5                   10                  15
   Ala Glu Leu Arg Gly Cys Val Asn Asp Val Arg Arg Met Arg Ile Ser
               20                  25                  30
   Leu Val Glu Arg Tyr Gly Phe Ser Glu Glu Asn Ile Lys Met Leu Ile
           35                  40                  45
   Asp Thr Asp Ser Ser Ser Ile Lys Pro Thr Gly Lys Asn Ile Arg Gln
       50                  55                  60
   Ala Leu Leu Asp Leu Val Glu Pro Ala Lys Ser Gly Asp Val Leu Phe
   65                  70                  75                  80
   Val His Tyr Ser Gly His Gly Thr Arg Leu Pro Ala Glu Thr Gly Glu
                   85                  90                  95
   Asp Asp Asp Thr Gly Tyr Asp Glu Cys Ile Val Pro Ser Asp Met Asn
               100                 105                 110
   Leu Ile Thr Asp Asp Asp Phe Arg Asp Leu Val Asp Met Val Pro Lys
           115                 120                 125
   Asp Cys Pro Ile Thr Ile Ile Ser Asp Ser Cys His Ser Gly Gly Leu
       130                 135                 140
   Ile Asp Glu Ala Lys Glu Gln Ile Gly Glu Ser Thr Lys Lys Lys Lys
   145                 150                 155                 160
   Asp Ser Gly Asp Ser Ser Thr Ile Asn Lys Glu Thr Glu Ala Glu Ile
                   165                 170                 175
   Ile Glu Val Gly Asn Arg Ser Leu Pro Leu Glu Thr Leu Ile Asp Met
               180                 185                 190
   Leu Lys Gln Glu Thr Gly Asn Asp Asp Ile Glu Val Gly Lys Ile Arg
           195                 200                 205
   Thr Thr Leu Phe Asp Met Phe Gly Asp Asp Ser Ser Pro Lys Val Lys
       210                 215                 220
   Lys Phe Met Asn Val Ile Leu Ser Asn Leu Gln Glu Thr Thr Thr Thr
   225                 230                 235                 240
   Ile Gln Thr Val Ser Asp Glu Val Leu Gly Ser Val Glu Asn Leu Ala
                   245                 250                 255
   Gln Glu Phe Leu Glu Gln Lys Leu Ser Asp Asp Val Lys Pro Ala Ile
               260                 265                 270
   Gln Asp Val Tyr Ala Gly Ala Ile Asn Gly Ala Leu Pro Asp Asn Gly
   275 280 285
   Ile Leu Ile Ser Gly Cys Gln Thr Asp Gln Thr Ser Ser Asp Ala Ser
   290 295 300
   Pro Pro Gly His Pro Glu Leu Ala Tyr Gly Ala Leu Thr Asn Ala Ile
   305 310 315 320
   Gln Ile Ile Ile Gly Glu Thr Lys Gly Lys Ile Ser Asn Lys Asp Leu
   325 330 335
   Val Leu Lys Ala Arg Lys Leu Leu Arg Lys Gln Gly Phe Asp Gln Arg
   340 345 350
   Pro Gly Leu Tyr Cys Asn Asp Ala Tyr Val Asn Ala Arg Phe Ile Cys
   355 360 365
<210> 6
   <211> 403
   <212> PRT
   <213> Arabidopsis thaliana
<220>
   <221> MISC_FEATURE
   <223> Atmc7
<400> 6
   Met Ala Lys Arg Ala Leu Leu Ile Gly Ile Asn Tyr Pro Gly Thr Thr
   1 5 10 15
   Glu Glu Leu Gln Gly Cys Val Asn Asp Val His Arg Met His Lys Cys
   20 25 30
   Leu Val Asp Arg Phe Gly Phe Ala Glu Glu Asp Ile Thr Val Leu Ile
   35 40 45
   Asp Thr Asp Glu Ser Tyr Thr Gln Pro Thr Gly Lys Asn Ile Arg Gln
   50 55 60
   Ala Leu Ser Glu Leu Ile Lys Pro Ala Lys Ser Gly Asp Val Leu Phe
   65 70 75 80
   Val His Tyr Ser Gly His Gly Thr Arg Val Pro Pro Glu Thr Gly Glu
   85 90 95
   Glu Asp Asp Thr Gly Phe Asp Glu Cys Ile Val Pro Ser Asp Leu Asn
   100 105 110
   Pro Ile Pro Asp Asp Asp Phe Arg Asp Leu Val Glu Gln Val Pro Glu
   115 120 125
   Gly Cys Gln Ile Thr Ile Val Ser Asp Ser Cys His Ser Gly Gly Leu
       130                 135                 140
   Ile Asp Glu Ala Lys Glu Gln Ile Gly Glu Ser Thr Thr Thr Lys Pro
   145                 150                 155                 160
   Asn Arg Glu Ser Lys Val Ser Ser Phe Glu Phe Glu Phe Lys Asn Cys
                   165                 170                 175
   Leu His Ser Ile Phe Val Lys Leu Leu Ala Phe Cys Gly Ile Gly Ser
               180                 185                 190
   Ser His Val Glu Thr Arg Glu Ile Val Glu Val Gly Glu Gly Asp Glu
           195                 200                 205
   Val Val Arg Ser Arg Tyr Leu Pro Leu Glu Arg Phe Ile Glu Leu Leu
       210                 215                 220
   Lys Gln Gln Thr Gly Gln Asp Asn Ile Glu Ile Gly Lys Ile Arg Pro
   225                 230                 235                 240
   Thr Leu Phe Asp Val Phe Gly Glu Asp Ser Ser Pro Lys Ile Lys Lys
                   245                 250                 255
   Phe Met Lys Val Ile Leu Thr Lys Leu Arg Lys Thr Asn Asp Gln Ser
               260                 265                 270
   Thr Leu Leu Gly Lys Ile Glu Glu Ser Ala Arg Gly Tyr Ile Glu Glu
           275                 280                 285
   Thr Leu Asn Asp Glu His Tyr Met Lys Pro Ala Met Gln Ala Gln Val
       290                 295                 300
   Lys Ser Asp Arg Glu Ile Tyr Gly Gly Arg Ser Ser Asn Gly Leu Phe
   305                 310                 315                 320
   Pro Asp Arg Gly Ile Leu Leu Ser Gly Cys Gln Thr Asp Glu Thr Ser
                   325                 330                 335
   Ala Asp Val Lys Lys Lys Gly Glu Ala Phe Gly Ala Phe Ser Asn Ala
               340                 345                 350
   Ile Gln Met Val Leu Ser Glu Thr Asp His Lys Asp Lys Ile Thr Asn
           355                 360                 365
   Lys Glu Met Val Leu Arg Ala Arg Glu Ile Leu Lys Lys Gln Met Phe
       370                 375                 380
   Ile Gln Arg Pro Gly Leu Tyr Cys Asn Asp Arg Phe Val Asn Ala Pro
   385                 390                 395                 400
   Phe Ile Cys
<210> 7
   <211> 381
   <212> PRT
   <213> Arabidopsis thaliana
<220>
   <221> MIST_FEATURE
   <223> Atmc8
<400> 7
   Met Ala Lys Lys Ala Leu Leu Ile Gly Ile Asn Tyr Pro Gly Thr Ala
   1               5                   10                  15
   Val Glu Leu Arg Gly Cys Val Asn Asp Val His Arg Met Gln Lys Cys
               20                  25                  30
   Leu Ile Glu Leu Tyr Gly Phe Ala Asn Lys Asp Ile Val Ile Met Ile
           35                  40                  45
   Asp Thr Asp Lys Ser Cys Ile Gln Pro Thr Gly Lys Asn Ile Cys Asp
       50                  55                  60
   Glu Leu Asp Asn Leu Ile Ala Ser Gly Gln Ser Gly Asp Phe Leu Val
   65                  70                  75                  80
   Phe His Tyr Ser Gly His Gly Thr Arg Ile Pro Pro Gly Ile Glu Asp
                   85                  90                  95
   Ser Glu Asp Pro Thr Gly Phe Asp Glu Cys Ile Thr Pro Cys Asp Met
               100                 105                 110
   Asn Leu Ile Lys Asp Gln Gln Phe Arg Glu Met Val Ser Arg Val Lys
           115                 120                 125
   Glu Gly Cys Gln Leu Thr Ile Ile Ser Asp Ser Cys His Ser Gly Gly
       130                 135                 140
   Leu Ile Gln Glu Val Lys Glu Gln Ile Gly Glu Ser His Met Lys Pro
   145                 150                 155                 160
   Val Asp Lys Val Lys Glu Gln Ile Glu Glu Ser His Met Lys Gln Pro
                   165                 170                 175
   Lys Leu Gly Ile Ala Ser Tyr Phe Leu Asn Ile Val Met Asn Leu Leu
               180                 185                 190
   Ala Thr Cys Gly Val Ser Lys Ser Gln Arg Asp Arg Gly Gly Gly Glu
           195                 200                 205
   Glu Ser Phe Arg Gly Glu Ile Glu Leu Glu Lys Asp Glu Thr Leu Asp
       210                 215                 220
   Ile Lys Thr Arg Tyr Leu Pro Phe Glu Ser Tyr Leu Ser Leu Leu Lys
   225                 230                 235                 240
   Glu Gln Thr Gly Gln Thr Asn Ile Glu Pro Val Arg Ile Arg Gln Thr
                   245                 250                 255
   Leu Leu Lys Leu Phe Gly Glu Asp Pro Ser Pro Asn Arg Gln Arg Gly
               260                 265                 270
   Leu Ser Asp Leu Gly Asn Cys Glu Val Asp Ala Gly Asp Ser Gly Ala
           275                 280                 285
   Ser Arg Leu Asn Ala Val Thr Asp Asn Gly Ile Leu Leu Ser Gly Cys
       290                 295                 300
   Gln Thr Asp Gln Arg Ser Glu Asp Val Tyr Val Thr Arg Thr Gly Lys
   305                 310                 315                 320
   Ala Tyr Gly Ala Phe Ser Asp Ala Ile Gln Met Ile Leu Ser Ala Pro
                   325                 330                 335
   Arg Lys Asp Lys Lys Lys Ile Thr Asn Lys Glu Leu Val Ser Glu Ala
               340                 345                 350
   Arg Val Phe Leu Lys Lys Arg Gly Tyr Ser Gln Arg Pro Gly Leu Tyr
           355                 360                 365
   Cys His Asp Arg Phe Val Asp Lys Pro Phe Ile Cys Tyr
       370                 375                 380
<210> 8
   <211> 6
   <212> PRT
   <213> Arabidopsis thaliana
<220>
   <221> misc_feature
   <222> (2)..(2)
   <223> x can be Y or F
<400> 8
   His Xaa Ser Gly His Gly
   1               5
<210> 9
   <211> 6
   <212> PRT
   <213> Arabidopsis thaliana
<220>
   <221> misc_feature
   <222> (2)..(2)
   <223> x can be A or S
<220>
   <221> misc_feature
   <222> (4)..(4)
   <223> x can be H or N
<400> 9
   Asp Xaa Cys Xaa Ser Gly
   1 5
<210> 10
   <211> 7
   <212> PRT
   <213> Arabidopsis thaliana
<400> 10
   His Tyr Ser Gly His Gly Thr
   1 5
<210> 11
   <211> 10
   <212> PRT
   <213> Arabidopsis thaliana
<400> 11
   Asp Ser Cys His Ser Gly Gly Leu Ile Asp
   1 5 10
<210> 12
   <211> 17
   <212> DNA
   <213> Artificial Sequence
<220>
   <223> Forward primer for Atmc1
<400> 12
   atgtacccgc cacctcc 17
<210> 13
   <211> 24
   <212> DNA
   <213> Artificial Sequence
<220>
   <223> Reverse primer for Atmc1
   <400> 13
   ctagagagtg aaaggctttg cata 24
<210> 14
   <211> 20
   <212> DNA
   <213> Artificial Sequence
<220>
   <223> Forward primer for Atmc2
<400> 14
   atgttgttgc tggtggactg 20
<210> 15
   <211> 27
   <212> DNA
   <213> Artificial Sequence
<220>
   <223> Reverse primer for Atmc2
<400> 15
   ttataaagag aagggcttct catatac 27
<210> 16
   <211> 19
   <212> DNA
   <213> Artificial Sequence
<220>
   <223> Forward primer for Atmc3
   <400> 16
   atggctagtc ggagagaag 19
<210> 17
   <211> 23
   <212> DNA
   <213> Artificial Sequence
<220>
   <223> Reverse primer for Atmc3
<400> 17
   tcagagtaca aactttgtcg cgt 23
<210> 18
   <211> 21
   <212> DNA
   <213> Artificial Sequence
<220>
   <223> Forward primer for Atmc4
<400> 18
   atgacgaaaa aggcggtgct t 21
<210> 19
   <211> 23
   <212> DNA
   <213> Artificial Sequence
<220>
   <223> Reverse primer for Atmc4
<400> 19
   tcaacagatg aaaggagcgt tgg 23
<210> 20
   <211> 21
   <212> DNA
   <213> Artificial Sequence
<220>
   <223> Forward primer for Atmc5
<400> 20
   atggcgaaga aagctgtgtt g 21
<210> 21
   <211> 24
   <212> DNA
   <213> Artificial Sequence
<220>
   <223> Reverse primer for Atmc5
<400> 21
   ttaacaaata aacggagcat tcac 24
<210> 22
   <211> 21
   <212> DNA
   <213> Artificial Sequence
<220>
   <223> Forward primer for Atmc6
<400> 22
   atggccaaga aagctttact g 21
<210> 23
   <211> 24
   <212> DNA
   <213> Artificial Sequence
<220>
   <223> Reverse primer for Atmc6
<400> 23
   tcaacatata aaccgagcat tgac 24
<210> 24
   <211> 21
   <212> DNA
   <213> Artificial Sequence
<220>
   <223> Forward primer for Atmc7
<400> 24
   atggcaaaga gagcgttgtt g 21
<210> 25
   <211> 24
   <212> DNA
   <213> Artificial Sequence
<220>
   <223> Reverse primer for Atmc7
<400> 25
   ttagcatata aacggagcat tcac 24
<210> 26
   <211> 21
   <212> DNA
   <213> Artificial Sequence
<220>
   <223> Forward primer for Atmc8
<400> 26
   atggcgaaga aagcactttt g 21
<210> 27
   <211> 27
   <212> DNA
   <213> Artificial Sequence
<220>
   <223> Reverse primer for Atmc8
<400> 27
   ttagtagcat ataaatggtt tatcaac 27
<210> 28
   <211> 21
   <212> DNA
   <213> Artificial Sequence
<220>
   <223> Forward primer for Atmc9
<400> 28
   atggatcaac aagggatggt c 21
<210> 29
   <211> 22
   <212> DNA
   <213> Artificial Sequence
<220>
   <223> Reverse primer for Atmc9
<400> 29
   tcaaggttga gaaaggaacg tc 22
<210> 30
   <211> 17
   <212> DNA
   <213> Artificial Sequence
<220>
   <223> Extension for forward primers
<400> 30
   aaaaagcagg ctccacc 17
<210> 31
   <211> 13
   <212> DNA
   <213> Artificial Sequence
<220>
   <223> Extension for reverse primers
<400> 31
   agaaagctgg gtc 13
<210> 32
   <211> 5
   <212> PRT
   <213> Artificial Sequence
<220>
   <223> Alignment group
<400> 32
   Pro Ala Gly Ser Thr
   1 5
<210> 33
   <211> 4
   <212> PRT
   <213> Artificial Sequence
<220>
   <223> Alignment group
<400> 33
   Gln Asn Glu Asp
   1
<210> 34
   <211> 4
   <212> PRT
   <213> Artificial Sequence
<220>
   <223> Alignment group
<400> 34
   Val Leu Ile Met
   1
<210> 35
   <211> 12
   <212> PRT
   <213> Artificial Sequence
<220>
   <223> Alignment group
<400> 35
   Pro Ala Gly Ser Thr Gln Asn Glu Asp His Lys Arg
   1               5                   10
<210> 36
   <211> 8
   <212> PRT
   <213> Artificial Sequence
<220>
   <223> Alignment group
<400> 36
   Cys Val Leu Ile Met Phe Tyr Trp
   1               5
<210> 37
   <211> 23
   <212> PRT
   <213> Artificial Sequence
<220>
   <223> N-terminal addition sequence
<400> 37
   Met Ser Tyr Tyr His His His His His His Leu Glu Ser Thr Ser Leu
   1               5                   10                  15
   Tyr Lys Lys Ala Gly Ser Thr
               20
<210> 38
   <211> 7
   <212> PRT
   <213> Artificial Sequence
<220>
   <223> Result of degradation p10-like fragment
<400> 38
   Ala Leu Pro Phe Lys Ala Val
   1 5
<210> 39
   <211> 24
   <212> PRT
   <213> Artificial Sequence
<220>
   <223> Short linker between HIS6-tag and Atmc9
<400> 39
   Met Ser Tyr Tyr His His His His His His Leu Glu Ser Thr Ser Leu
   1 5 10 15
   Tyr Lys Lys Ala Gly Ser Thr Met
   20
<210> 40
   <211> 367
   <212> PRT
   <213> Arabidopsis thaliana
<220>
   <221> MIST-FEATURE
   <223> Atmc1
<400> 40
   Met Tyr Pro Pro Pro Pro Ser Ser Ile Tyr Ala Pro Pro Met Leu Val
   1 5 10 15
   Asn Cys Ser Gly Cys Arg Thr Pro Leu Gln Leu Pro Ser Gly Ala Arg
               20                  25                  30
   Ser Ile Arg Cys Ala Leu Cys Gln Ala Val Thr His Ile Ala Asp Pro
           35                  40                  45
   Arg Thr Ala Pro Pro Pro Gln Pro Ser Ser Ala Pro Ser Pro Pro Pro
       50                  55                  60
   Gln Ile His Ala Pro Pro Gly Gln Leu Pro His Pro His Gly Arg Lys
   65                  70                  75                  80
   Arg Ala Val Ile Cys Gly Ile Ser Tyr Arg Phe Ser Arg His Glu Leu
                   85                  90                  95
   Lys Gly Cys Ile Asn Asp Ala Lys Cys Met Arg His Leu Leu Ile Asn
               100                 105                 110
   Lys Phe Lys Phe Ser Pro Asp Ser Ile Leu Met Leu Thr Glu Glu Glu
           115                 120                 125
   Thr Asp Pro Tyr Arg Ile Pro Thr Lys Gln Asn Met Arg Met Ala Leu
       130                 135                 140
   Tyr Trp Leu Val Gln Gly Cys Thr Ala Gly Asp Ser Leu Val Phe His
   145                 150                 155                 160
   Tyr Ser Gly His Gly Ser Arg Gln Arg Asn Tyr Asn Gly Asp Glu Val
                   165                 170                 175
   Asp Gly Tyr Asp Glu Thr Leu Cys Pro Leu Asp Phe Glu Thr Gln Gly
               180                 185                 190
   Met Ile Val Asp Asp Glu Ile Asn Ala Thr Ile Val Arg Pro Leu Pro
           195                 200                 205
   His Gly Val Lys Leu His Ser Ile Ile Asp Ala Cys His Ser Gly Thr
       210                 215                 220
   Val Leu Asp Leu Pro Phe Leu Cys Arg Met Asn Arg Ala Gly Gln Tyr
   225                 230                 235                 240
   Val Trp Glu Asp His Arg Pro Arg Ser Gly Leu Trp Lys Gly Thr Ala
                   245                 250                 255
   Gly Gly Glu Ala Ile Ser Ile Ser Gly Cys Asp Asp Asp Gln Thr Ser
               260                 265                 270
   Ala Asp Thr Ser Ala Leu Ser Lys Ile Thr Ser Thr Gly Ala Met Thr
           275                 280                 285
   Phe Cys Phe Ile Gln Ala Ile Glu Arg Ser Ala Gln Gly Thr Thr Tyr
       290                 295                 300
   Gly Ser Leu Leu Asn Ser Met Arg Thr Thr Ile Arg Asn Thr Gly Asn
   305                 310                 315                 320
   Asp Gly Gly Gly Ser Gly Gly Val Val Thr Thr Val Leu Ser Met Leu
   325 330 335
   Leu Thr Gly Gly Ser Ala Ile Gly Gly Leu Arg Gln Glu Pro Gln Leu
   340 345 350
   Thr Ala Cys Gln Thr Phe Asp Val Tyr Ala Lys Pro Phe Thr Leu
   355 360 365
<210> 41
   <211> 418
   <212> PRT
   <213> Arabidopsis thaliana
<220>
   <221> MISC_FEATURE
   <223> Atmc2
<400> 41
   Met Leu Leu Leu Val Asp Cys Ser Ser Cys Arg Thr Pro Leu His Leu
   1 5 10 15
   Pro Pro Gly Ala Thr Arg Ile Arg Cys Ala Ile Cys His Ala Phe Thr
   20 25 30
   Leu Ile Ala Pro Glu Pro Arg Leu Gln Ser His Ala Ser Ala Ser Pro
   35 40 45
   Phe Pro Phe Pro Asn Ser Ser Pro Ala Pro Ser Thr Phe Ile Tyr Pro
   50 55 60
   Pro Pro Thr Pro Ser Pro Tyr Thr His Ala Pro His Ala Pro Ser Pro
   65 70 75 80
   Phe Asn His Ala Pro Pro Asp Ser Tyr Pro Phe Thr His Ala Pro Pro
   85 90 95
   Ala Ser Ser Pro Phe Asn His Ala Pro Pro Gly Pro Pro Pro Pro Val
   100 105 110
   His Gly Gln Lys Arg Ala Val Ile Val Gly Val Ser Tyr Lys Asn Thr
   115 120 125
   Lys Asp Glu Leu Lys Gly Cys Ile Asn Asp Ala Asn Cys Met Lys Phe
   130 135 140
   Met Leu Met Lys Arg Phe Gln Phe Pro Glu Ser Cys Ile Leu Met Leu
   145 150 155 160
   Thr Glu Glu Glu Ala Asp Pro Met Arg Trp Pro Thr Lys Asn Asn Ile
   165 170 175
   Thr Met Ala Met His Trp Leu Val Leu Ser Cys Lys Pro Gly Asp Ser
               180                 185                 190
   Leu Val Phe His Phe Ser Gly His Gly Asn Asn Gln Met Asp Asp Asn
           195                 200                 205
   Gly Asp Glu Val Asp Gly Phe Asp Glu Thr Leu Leu Pro Val Asp His
       210                 215                 220
   Arg Thr Ser Gly Val Ile Val Asp Asp Glu Ile Asn Ala Thr Ile Val
   225                 230                 235                 240
   Arg Pro Leu Pro Tyr Gly Val Lys Leu His Ala Ile Val Asp Ala Cys
                   245                 250                 255
   His Ser Gly Thr Val Met Asp Leu Pro Tyr Leu Cys Arg Met Asp Arg
               260                 265                 270
   Leu Gly Asn Tyr Glu Trp Glu Asp His Arg Pro Lys Thr Gly Met Trp
           275                 280                 285
   Lys Gly Thr Ser Gly Gly Glu Val Phe Ser Phe Thr Gly Cys Asp Asp
       290                 295                 300
   Asp Gln Thr Ser Ala Asp Thr Pro Gln Leu Ser Gly Ser Ala Trp Thr
   305                 310                 315                 320
   Gly Ala Met Thr Tyr Ala Phe Ile Gln Ala Ile Glu Arg Gly His Gly
                   325                 330                 335
   Met Thr Tyr Gly Ser Leu Leu Asn Ala Met Arg Ser Thr Val His Glu
               340                 345                 350
   Ile Phe Asp Lys Asn Lys Gly Arg Glu Leu Val Glu Val Gly Gly Ala
           355                 360                 365
   Asp Phe Leu Ser Thr Leu Leu Gly Leu Leu Ile Leu Gly Ala Ser Pro
       370                 375                 380
   Pro Asp Glu Glu Glu Glu Val Asn Gln Ala Pro Gln Lys Thr Gln Glu
   385                 390                 395                 400
   Pro Gln Leu Ser Ala Asn Glu Ala Phe Ala Val Tyr Glu Lys Pro Phe
                   405                 410                 415
   Ser Leu
<210> 42
   <211> 362
   <212> PRT
   <213> Arabidopsis thaliana
<220>
   <221> MIST_FEATURE
   <223> Atmc3
<400> 42
   Met Ala Ser Arg Arg Glu Val Arg Cys Arg Cys Gly Arg Arg Met Trp
   1 5 10 15
   Val Gln Pro Asp Ala Arg Thr Val Gln Cys Ser Thr Cys His Thr Val
   20 25 30
   Thr Gln Leu Tyr Ser Leu Val Asp Ile Ala Arg Gly Ala Asn Arg Ile
   35 40 45
   Ile His Gly Phe Gln Gln Leu Leu Arg Gln His Gln Pro Gln His His
   50 55 60
   Glu Gln Gln Gln Gln Gln Met Met Ala Gln Pro Pro Pro Arg Leu Leu
   65 70 75 80
   Glu Pro Leu Pro Ser Pro Phe Gly Lys Lys Arg Ala Val Leu Cys Gly
   85 90 95
   Val Asn Tyr Lys Gly Lys Ser Tyr Ser Leu Lys Gly Cys Ile Ser Asp
   100 105 110
   Ala Lys Ser Met Arg Ser Leu Leu Val Gln Gln Met Gly Phe Pro Ile
   115 120 125
   Asp Ser Ile Leu Met Leu Thr Glu Asp Glu Ala Ser Pro Gln Arg Ile
   130 135 140
   Pro Thr Lys Arg Asn Ile Lys Lys Ala Met Arg Trp Val Ile Glu Gly
   145 150 155 160
   Asn Arg Ala Arg Asp Ser Leu Val Phe His Phe Ser Gly His Gly Ser
   165 170 175
   Glu Gln Asn Asp Tyr Asn Gly Asp Glu Ile Asp Gly Gln Asp Glu Ala
   180 185 190
   Leu Cys Pro Leu Asp His Glu Thr Glu Gly Lys Ile Ile Asp Asp Glu
   195 200 205
   Ile Asn Arg Ile Leu Val Arg Pro Leu Val His Gly Ala Lys Leu His
   210 215 220
   Ala Val Ile Asp Ala Cys Asn Ser Gly Thr Val Leu Asp Leu Pro Phe
   225 230 235 240
   Ile Cys Arg Met Glu Arg Asn Gly Ser Tyr Glu Trp Glu Asp His Arg
   245 250 255
   Ser Val Arg Ala Tyr Lys Gly Thr Asp Gly Gly Ala Ala Phe Cys Phe
   260 265 270
   Ser Ala Cys Asp Asp Asp Glu Ser Ser Gly Tyr Thr Pro Val Phe Thr
           275                 280                 285
   Gly Lys Asn Thr Gly Ala Met Thr Tyr Ser Phe Ile Lys Ala Val Lys
       290                 295                 300
   Thr Ala Gly Pro Ala Pro Thr Tyr Gly His Leu Leu Asn Leu Met Cys
   305                 310                 315                 320
   Ser Ala Ile Arg Glu Ala Gln Ser Arg Leu Ala Phe Asn Gly Asp Tyr
                   325                 330                 335
   Thr Ser Ser Asp Ala Ser Ala Glu Pro Leu Leu Thr Ser Ser Glu Glu
               340                 345                 350
   Phe Asp Val Tyr Ala Thr Lys Phe Val Leu
           355                 360
<210> 43
   <211> 24
   <212> DNA
   <213> Artificial Sequence
<220>
   <223> Forward primer for generation of prodomain deletion mutant
<400> 43
   atggcagttt tatgcggcgt gaac 24
<210> 44
   <211> 34
   <212> DNA
   <213> Artificial Sequence
<220>
   <223> Forward primer for Gateway cloning
<400> 44
   ggggacaagt ttgtacaaaa aagcaggctc cacc 34
<210> 45
   <211> 30
   <212> DNA
   <213> Artificial Sequence
<220>
   <223> Reverse primer for Gateway cloning
<400> 45
   ggggaccact ttgtacaaga aagctgggtc 30

## Description

The present invention relates to a novel class of metacaspases. More particularly, the present invention relates to the use of metacaspases, preferably plant metacaspases to process a protein at a cleavage site comprising an arginine or a lysine at the P1 position, and to the use of such metacaspases to modulate cell death.

Cell death is a certitude for every living bacterial, unicellular, or multicellular organism. In what is generally called programmed cell death (PCD), this is triggered by extracellular or intracellular signals, and it is associated with development and environmental stress. In animals, cell death occurs mainly in development, tissue remodeling, and immune regulation, but it is also involved in many pathologies (Ellis *et al*., 1991; Williams, 1994). Based on primarily morphological features, animal cell death is usually referred to as apoptosis or necrosis. Apoptosis is characterized by membrane blebbing, cytosolic condensation, cell shrinkage, nuclear condensation, breakdown of nuclear DNA (DNA laddering), and finally the formation of apoptotic bodies, which can easily be taken up by other cells (Fiers *et al.,* 1999). Necrosis, as defined on a microscopic level, denotes cell death where cells swell, round up, and then suddenly collapse, spilling their contents in the medium. However, in animals other forms of cell death exist, like autophagic and autolytic death, and it is now gradually accepted that all intermediate varieties of cell death can occur (Lockshin and Zakeri, 2002).

Also in plants, cell death is a prerequisite process in development, morphogenesis, maintenance and reproduction (Greenberg, 1996; Pennell and Lamb, 1997; Buckner *et al*., 2000). During reproductive development, cell death is involved in a plethora of processes like pollen grain production, female gametophyte formation, pollination, and embryogenesis (Wu and Cheun, 2000). In cereals, formation of the starchy endosperm requires apoptosis-like cell death, while the cells of the aleurone layer die a few days after germination through a rather autolytic process (Young and Gallie, 2000; Fath *et al.,* 2000). During growth of a plant, formation of tracheary elements from procambium, as experimentally represented by differentiating *Zinnia* cells, relies on a type of cell death that is characterized by vacuolar collapse, a process which is probably orchestrated by the mitochondria (Yu *et al*., 2002; Fukuda, 2000). Especially important for worldwide agriculture is cell death as part of the hypersensitive response (HR) of plants to pathogens (for reviews, see ref. Greenber, 1997; Heath, 2000; Morel and Dangl, 1997). The HR is a rapid process, mainly characterized by the appearance of small lesions at the site of pathogen infection, a plant-directed strategy which is that of the "scorched earth". Thus, the death of plant cells at the site of infection is deleterious for pathogens, at least for so-called obligate biotrophic ones.

In plants, PCD or "active cell death" are terms usually applied to denote apoptosis-like cell death, showing features like chromatin aggregation, cell shrinkage, cytoplasmic and nuclear condensation and DNA fragmentation (Buckner *et al*., 2000;Jabs, 1999; O'Brien *et al*., 1998).

Apoptotic characteristics have been observed during HR and following abiotic stress, such as ozone, UV irradiation, chilling and salt stress (Pennell and Lamb, 1997; Danon and Gallois, 1998; Katsuhara, 1997; Kratsch and Wise, 2000; Pellinen *et al*., 1999). Necrosis or "passive cell death" is used to describe cell death that results from severe trauma during exterme stress situations and occurs immediately and independently of any cellular activity (O'Brien et a/., 1998).

On a biochemical level, apoptosis in animals is characterized, and commonly also defined, by the activation of a distinct family of cysteine-dependent aspartate-specific proteases or caspases (Eamshaw *et al.,* 1999). Mature active caspases are derived from their zymogen by proteolysis at specific aspartate residues, removing an N-terminal prodomain and separating the large (p20) and small (p10) subunits, two of each forms a fully active caspase enzyme.

The unprocessed forms of most caspases already possess low intrinsic protease activity.

Thus, during cell death signaling, the most upstream caspases, called initiator caspases, associate through their large prodomain with adaptor proteins and are forced to auto-process by what is known as induced proximity. The resulting fully active caspases are then able to proteolytically activate downstream executioner caspases. These are able to cut a variety of cellular substrates, resulting in a plethora of structural and metabolic alterations, ultimately leading to an organized death of the cell. (Eamshaw *et al*., 1999; Utz and Anderson, 2000; Cohen,1997).

Using synthetic oligopeptide caspase substrates and inhibitors, caspase-like activity could already be demonstrated in various plant cell death models. Co-infiltration in tobacco of caspase-inhibitors with an incompatible *Pseudomonas syringae* pathovar prevented HR (del Pozo and Lamb, 1998). Also, chemical-induced cell death in tomato cells could be blocked by addition of different caspase inhibitors (De Jong *et al*., 2000). Tobacco plants infected by the tobacco mosaic virus show protease activity as measured by Ac -YVAD-AMC, a synthetic substrate for caspase-1 (del Pozo and Lamb, 1998). When soybean cells are subjected to oxidative stress, cysteine proteases are activated, and Inhibition of some of these by cystatin almost completely blocked cell death (Solomon *et al*., 1999). Korthout and co-workers showed that embryonic barley cells contain caspase 3-like activity, as measured with the specific substrate acetyl-Asp-Glu-Vel-Asp-aminomethylcoumarin (Ac-DEVD-AMC) (Korthout *et al*., 2000). Recently, Uren *et al.* (2000) reported the existence of two families of distant caspase homologues in plants, fungi, protozoa and animals. Paracaspases are, like caspases, restricted to the animal kingdom, while metacaspases could be found in plants, fungi and protozoa. However, the existence of these metacaspases was only derived from *in silico* data, and the activity of the metacaspases has not been demonstrated. Moreover, it was clearly stated that it remained to be seen whether the stress-induced caspase activity in plants is exerted by the metacaspases, or by other unknown members of the caspase-like superfamily.

Szallies et al. (FEBS Letters 2002: 144) demonstrated that heterologous expression of Trypanosoma brucei metacaspase TbMCA4 in the budding yeast Saccharomyces cerevisiae resulted in growth inhibition, mitochondrial dysfunction and clonal death.

Database entry UNIPROT accession No. Q9FYE1 discloses a fragment of SEQ ID NO:1 of the present invention.

Surprisingly, we found a new member of this metacaspase family and determined its activity.

In contrast to the known caspases, that have a D residue at position P1, the novel metacaspase family is cutting after arginine and/or lysine, i.e. its recognition site has either an R or a K at position P1. Preferably, the novel metacaspase family is cutting after arginine.

Even more preferably, the novel metacaspase family is cutting after arginine and lysine.

A first aspect of the invention is the use of a metacaspase to process a protein at a cleavage site comprising arginine and/or lysine at position P1. Although there are proteases known, such as clostripain and gingipain that cut at a cleavage site with an R or K at position P1, those proteases are only distantly related and show no significant overall homology with the metacaspases described here. One preferred embodiment is a metacaspase according to the invention that is active at acidic pH. Preferably, said metacaspase shows it maximal activity at acidic pH, preferably in a ph range of 5-6, even more preferably in a pH range of 5.2-5.5.

Preferably the metacaspase used according to the invention is a plant metacaspase. Even more preferably, said metacaspase is selected from the group consisting of polypeptides comprising, SEQ ID N°2.

Most preferably, the metacaspase used according to the invention consists of SEQ ID N° 1,.

Another aspect of the invention is the use of a metacaspase, which is cleaving at a cleavage site comprising arginine and/or lysine at position P1, to modulate cell growth, preferably to modulate cell death, even more preferably to modulate programmed cell death. Preferably, said metacaspase is cutting after arginine. Even more preferably, said metacaspase is cutting after arginine and lysine. Preferably, said modulation of cell death is obtained in plant cells.

Preferably the metacaspase used according to the invention is a plant metacaspase. Even more preferably, said metacaspase is selected from the group consisting of polypeptides comprising, SEQ ID N°2.

Most preferably,the metacaspase used according to the invention consists of SEQ ID N° 1.

Said modulation can be an increase as well as a decrease of cell death. An increase of cell death can be obtained by overexpression of the metacaspase according to the invention; the effect of the metacaspase may be either direct, by degradation of essential proteins, or indirect, by activation of other proteases or lytic enzymes. An increase in cell death may be interesting, as a non-limiting example, incase of pathogen response, whereby the gene encoding the metacaspase is operably linked to a pathogen inducible promoter. Pathogen inducible promoters are known to the person skilled in the art, and have been disclosed, amongst others, in WO9950428, W00001830 and WO0060086. Alternatively, cell death may be wished to obtain tissue abortion, such as in the case of male sterility. In this case, the gene encoding the metacaspase can operably linked to a tissue specific promoter. Tissue specific promoters are also known to the person skilled in the art.

A decrease of cell death can be obtained by downregutation of the expression of the metacaspase, of by inhibition of its activity, inhibition of the activity can be realized in several ways. As non-limiting example, the self-processing can be blocked, e.g. by mutagenesis of the cleavage site. Alternatively, a specific inhibitor may be used. As a non-limiting example, a specific inhibitor may be an antibody that binds to the active site of the metacaspase, or an antibody that binds to the cleavage site of the substrate, or a peptide or peptidomimetic comprising the cleavage site.

### Definitions

Metacaspase as used here is a polypeptide with a proteolytic activity, comprising in its non-processed form the sequences H Y/F SGHG and D A/S C HIN SG. Preferably, the polypeptide comprises in its non-processed form the sequences HYSGHGT and DSCHSGGLID.

*Functional fragment* as used here means that the fragment Is essential for the metacaspase activity. However, it does not imply that the fragment on its own is sufficient for activity. Typical functional fragments for the type II metacaspases are the so-called p10 and p20-like fragments.

Typical functional fragments for the type I metacaspases are fragment where the so-called prodomain has been deleted. The metacaspase activity as defined here means the proteolytic activity, by which a protein is processed at a cleavage site comprising an arginine or lysine residue at position P1. Preferably, said metacaspase is cleaving after arginine. Even more preferably, said metacaspase is cleaving after arginine and lysine.

*Position P1* is the C-terminal residue of the fragment upstream of the cleavage site (the amino-terminal fragment).

*Derived from a plant* as used here means that the gene, encoding the metacaspase, was originally isolated from a plant. It doesn't imply that the metacaspase is produced in, or isolated from a plant. Indeed, the metacaspase may be produced in another host organism, such as a bacterium, whereby it is either isolated after production, or exerts its activity *in vivo* in the host.

*Operably linked* refers to a juxtaposition wherein the components so described are in a relationship permitting them to function in their intended manner. A promoter sequence "operably linked" to a coding sequence is ligated in such a way that expression of the coding sequence is achieved **under** conditions compatible with the promoter sequence.

An *acidic pH* as used here means a pH below pH 7, preferably below pH 6,5, even more preferably below ph 6. The most preferred range is between pH 5 and 6, even more preferred between 5.2 and 5.5. An *alkaline pH* as used here means a pH above pH 7, preferably above pH 7,5. The most preferred range is between pH 7.5 and 8.

### Brief description of the figures

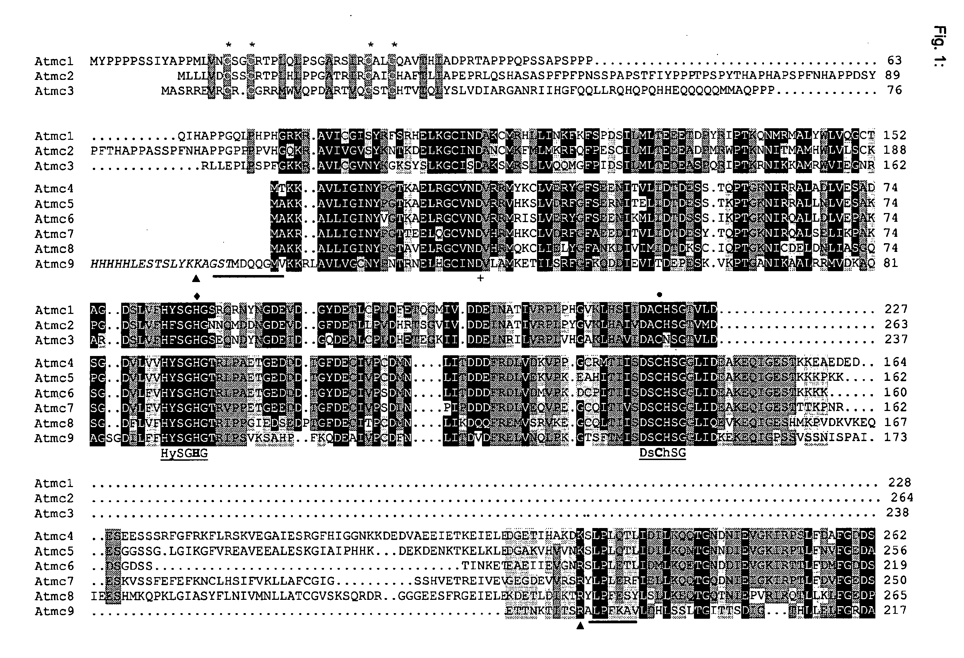

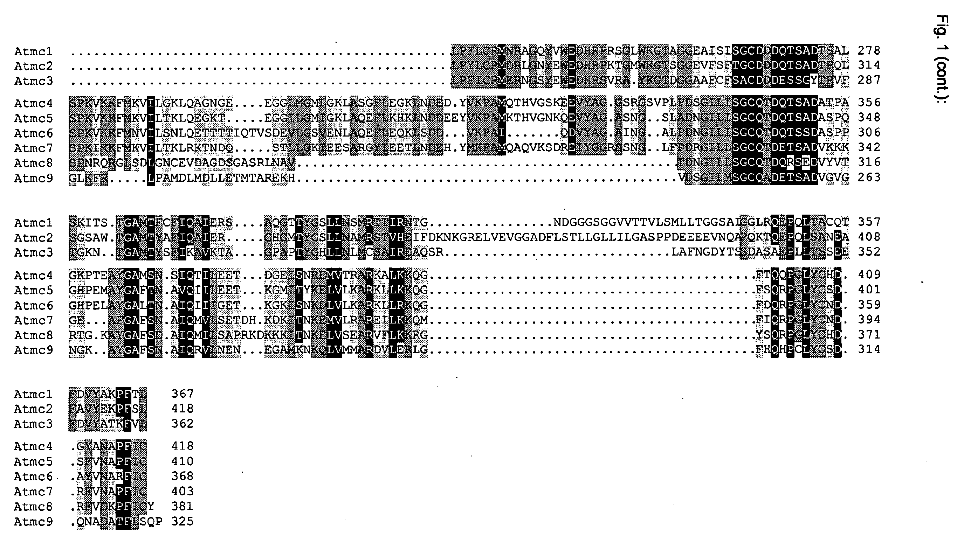

**Figure 1****:** The *Arabidopsis thaliana* metacaspase family. Multiple alignment of the nine metacaspases in *Arabidopsis thaliana.* For shading details, see materials and methods. The putative catalytic His and Cys residues are marked by a diamond and a dot, respectively, while their surrounding conserved residues are marked by a letter. Zinc finger cysteines in the prodomains of type I metacaspases are marked by an asterisk. The P1 positions for autocatalytic cleavage of Atmc9 are denoted by a triangle, while the obtained N-terminal peptide sequences for Atmc9 (shown with part of the N-terminal HIS₆-tag) are underlined. The aspartate residue possibly involved in coordination of the substrate P1 is marked by a +.

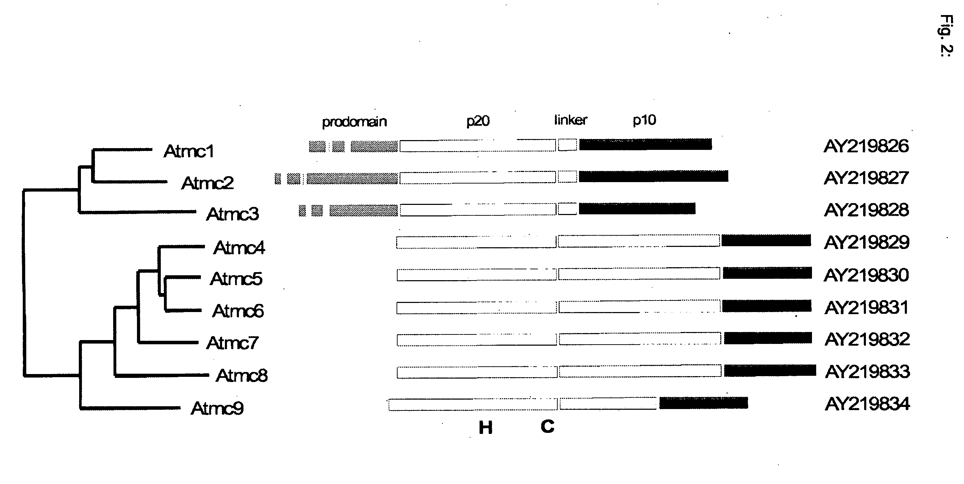

**Figure 2****:** Unrooted phylogenetic tree of the *Arabidopsis thaliana* metacaspase family. For construction of the tree, the alignment of Figure 1 was subjected to the TREECON software package (Van de Peer and De Wachter, 1994). On the right side a tentative schematic representation of the structure of the nine *Arabidopsis* metacaspases is shown. The putative prodomain is depicted in dark gray, the large subunit ("p20") in white, and the small subunit ("p10") in black. Linker regions between p20 and p10 are shown in light gray. Cysteine residues of the prodomain Zn-fingers are shown as white bars. Genbank accession numbers are also shown.

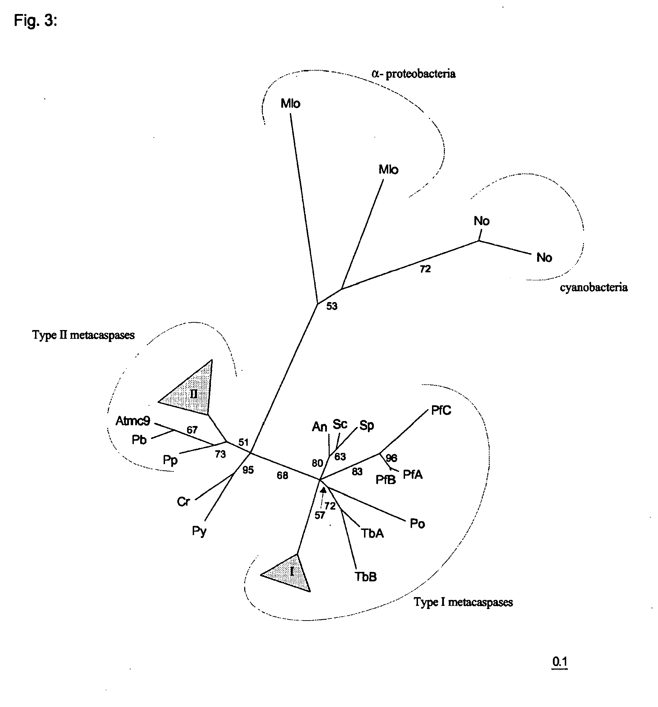

**Figure 3****:** Unrooted Maximum-Likehood phylogenetic tree of metacaspases on the region corresponding to the p20 subunit. Triangle I represents Atmc1-3, Tm, Ls, Ha, LeA and Sec, where triangle II represents Atmc4-9, Hb, LeB, Ha, Ga, Mt, Gm, Mc, Ro, Pd, Os, Cer and Pip. Abbreviations: An, *Aspergillus nidulans;* At, *Arabidopsis thaliana;* Cer, *Ceratopteris richardii;* Cr, *Chlamydomonas reinhardtii;* Ga, *Gossypium arboreum;* Gm, *Glycine max;* Ha, *Helianthus annuus;* Hb, *Hevea brasiliensis;* Le, *Lycopersicon esculentum;* Ls, *Lactuca sativa;* Mc, *Mesembryanthemum crystallinum;* Mt, *Medicago truncatula;* Mlo, *Mesorhizobium loti;* No, *Nostoc* sp. PCC 7120; Os, *Oryza sativa;* Pb, *Populus balsamifera;* Pd, *Prunus dulcis;* Pf, *Plasmodium falciparum;* Pip, *Pinus pinaster,* Po, *Pleurotus ostreatus;* Pp, *Physcomitrella patens;* Py, *Porphyra yezoensis;* Ro, Rosa hybrid cultivar; Sec, Secale *cereale;* Sc, *Saccharomyces cerevisiae;* Sp, *Schizosaccharomyces pombe;* Tb, *Trypanosoma brucei;* Tm, *Triticum monococcum.* The alignments are available from the authors upon request.

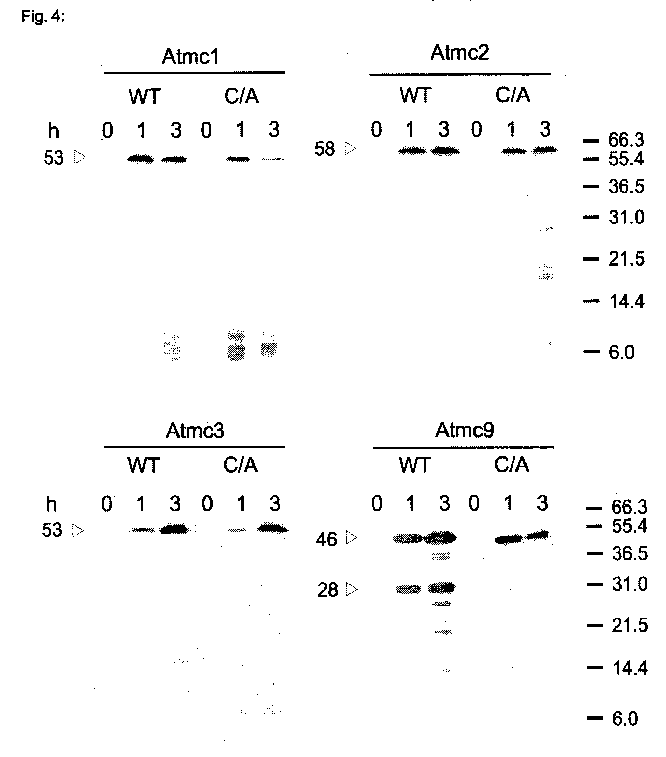

**Figure 4****:** Bacterial expression of *Arabidopsis* metaca spases. Bacterial cultures carrying an expression vector for N-terminally HIS₆-tagged Atmc1, -2, -3 and -9 wild-type or C/A were induced during 1 or 3 hours and whole lysates subjected to immunoblotting with anti-HIS.

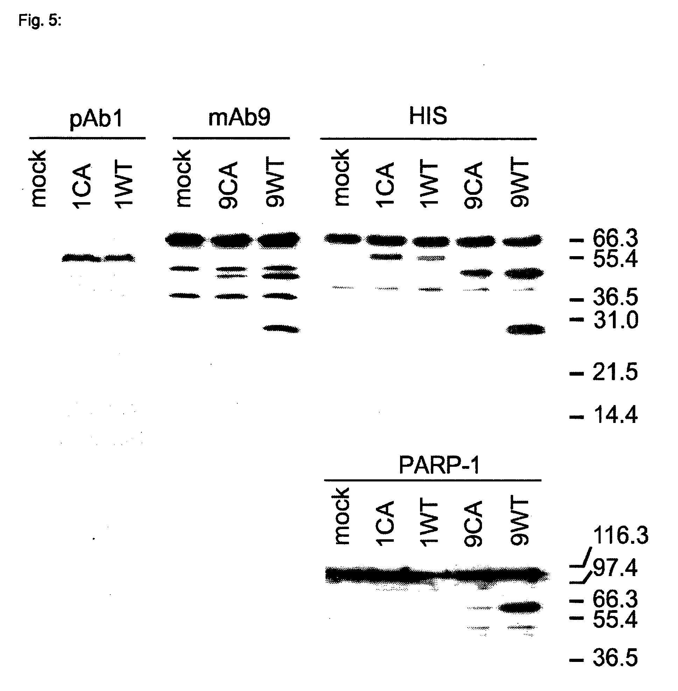

**Figure 5****:** Overexpression analysis of *Arabidopsis* metacaspases in human embryonic kidney 293T cells. Upper left: Overexpression of Atmc1 and detection with polyclonal antibodies. Lane 1, mock transfected; lane 2, C/A mutant; lane 3, wild-type. Upper middle: Overexpression of Atmc9 and detection with monoclonal antibodies. Lane 1, mock transfected; lane 2, C/A mutant; lane 3, wild-type. Upper right: Detection of Atmc1 and -9 with anti-HIS antibodies. Lane 1, mock transfected; lanes 2 and 3, Atmc1 C/A and wild-0type, resp.; lanes 4 and 5, Atmc9 C/A and wild-type, resp. Lower panel: Detection of human PARP-1. Lane 1, mock transfected; lanes 2 and 3, Atmc1 C/A and wild-type, resp.; lanes 4 and 5, Atmc9 C/A and wild-type, resp.

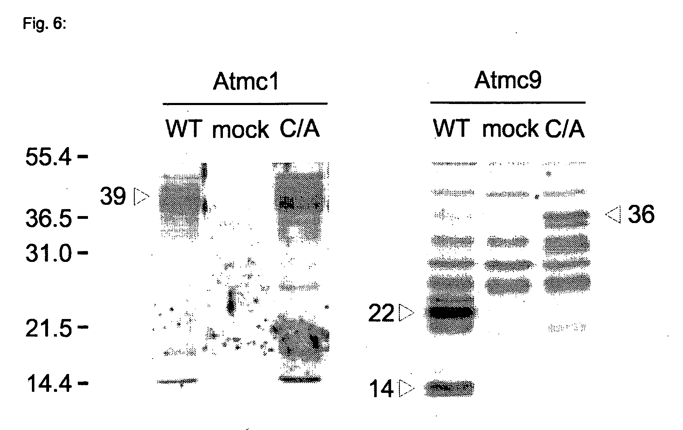

**Figure 6****:** Overexpression analysis of *Arabidopsis* metacaspases in *N. benthamiana.* Left panel: Overexpression of Atmc1 and detection with polyclonal antibodies. Lane 1, wild-type; lane 2, C/A mutant; lane 3, mock. Right panel: Overexpression of Atmc9 and detection with polyclonal antibodies. Lane 1, wild-type; lane 2, C/A mutant; lane 3, mock. and -9 and their respective C/A mutants in *N. benthamiana.*

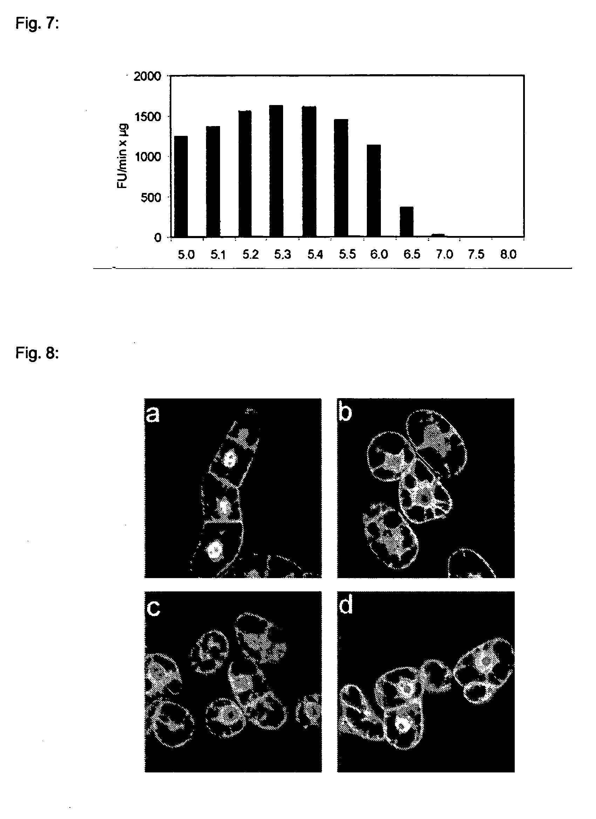

**Figure 7****:** Proteolytic activity of Atmc9 against Boo-GKR-AMC at different pH.
**Figure 8****:** Subcellular localization of C-terminal GFP fusions of *Arabidopsis* metacaspases in tobacco BY-2 cells. Panels (a) to (d) show confocal images of BY-2 cells overproducing GFP-fusions with Atmc1, Atmc2, Atmc3 and Atmc9, respectively.

### Examples

### Materials and methods too the examples

### Used databases for the detection of the genes for Arabidopsis metacaspases

A genome-wide, non-redundant collection of *Arabidopsis* protein-encoding genes was predicted with Gene-Mark.hmm (Lukashin and Borodovsky, 1998). Based on these predictions, searchable databases of virtual transcripts and corresponding protein sequences were generated.

### Cloning of Arabidopsis metacaspase ORF's

Total RNA was isolated from leaves, inflorescences and roots of young and mature plants. First strand cDNA was synthesized from pooled RNA using Superscript II RNase H- RT (Invitrogen, Gaithersburg, MD, USA) using the manufacturer's instructions, and used as template for PCR reactions using PLATINUM Pfx DNA polymerase (Invitrogen, Gaithersburg, MD, USA) and the following forward and reverse primers: Atmc1: 5'ATGTACCCGCCACCTCC3' and 5'CTAGAGAGTGAAAGGCTTTGCATA3'; Atmc2: 5'ATGTTGTTGCTGGTGGACTG3' and 5'TfATAAAGAGAAGGGCTTCTCATATAC3'; Atmc3: 5'ATGGCTAGTCGGAGAGAAG3' and 5'TCAGAGTACAAACTTTGTCGCGT3'; Atmc4: 5'ATGACGAAAAAGGCGGTGCTT3' and 5'TCAACAGATGAAAGGAGCGTTGG3'; Atmc5: 5'ATGGCGAAGAAAGCTGTGTTG3' and 5'TTAACAAATAAACGGAGCATTCAC3'; Atmc6: 5'ATGGCCAAGAAAGCTTTACTG3' and 5'TCAACATATAAACCGAGCATTGAC3'; Atmc7: 5'ATGGCAAAGAGAGCGTTGTTG3' and 5'TTAGCATATAAACGGAGCATTCAC3'; Atmc8: 5'ATGGCGAAGAAAGCACTTTTG3' and 5'TTAGTAGCATATAAATGGTTTATCAAC3'; Atmc9: 5'ATGGATCAACAAGGGATGGTC3' and 5'TCAAGGTTGAGAAAGGAACGTC3'. For forward primers, the following bases were attached to the 5' end to enable subsequent amplification with the attB1 primer: 5'AAAAAGCAGGCTCCACC3'. For reverse primers, the 5' extension was 5'AGAAAGCTGGGTC3' to allow annealing with attB2.

PCR products were purified using gel electrophoresis and used as template in a second PCR with attB1 and attB2 primers, to allow subsequent Gateway cloning procedures (Invitrogen). Products were purified on gel and cloned into pDONR201 to generate entry vectors for each metacaspase.

### Alignment of metacaspase sequences

Sequences were aligned using clustalX (Thompson *et al.,* 1997), and manually edited with BioEdit (Hall, 1999). For shading of the alignment, amino acid groups as described in (Wu and Brutlag, 1995) were used. Besides identical amino acids, those belonging to the following groups were scored as highly homologous: [PAGST], [QNED], [KRH], [VLIM] and [FYW], and are shaded black (all metacaspases) or dark gray (one type of metacaspases only) in the alignment. The following groups were used to determine weakly conserved residues: [PAGSTQNEDHKR] and [CVLIMFYW].

### Phylogeny

To determine metacaspase orthologues, a profile constructed with annotated eukaryotic metacaspase protein sequences was used to search the public protein databases (HMMer; Eddy, 1998). Protein sequences of putative metacaspases were aligned to the profile using CLUSTALW (Thompson *et al.,* 1994). Manual editing of the alignment was performed with BioEdit (Hall, 1999), reformatting using ForCon (Raes and Van de Peer, 1999). Phylogenetic trees were constructed with the Maximum-Likelihood program TREE-PUZZLE (Schmidt *et al.* 2002) and the Neighbour-Joining algorithm, implemented in TREECON (Van de Peer and De Wachter, 1994) on the region probably corresponding to the p20 subunit. Distance matrices were calculated based on the Poisson correction.

### Bacterial production and antibodies

The cDNAs for metacaspases were cloned into the bacterial expression vector pDEST17 (Invitrogen), resulting in N-terminal addition of the amino acid sequence MSYYHHHHHHLESTSLYKKAGST, and the plasmids were introduced into E. *coli* strain BL21(DE3). Bacterial cultures were induced with 1 mM IPTG for 1-3 hours, cells were spun down and lysed under denaturing conditions adapted from *Rogl et al.* (1998). Briefly, the bacterial cell pellet from a 0.5 l culture was lysed using 5 ml 100 mM Tis.Cl pH 8.0, 20 ml 8.0 M urea, and 2.7ml 10% sodium N-lauroyl-sarcosinate, completed with 1 mM PMSF and 1 mM oxidized gluthation. After sonication, the volume was brought to 50 ml with buffer 1 (20 mM Tris.Cl pH 8.0, 200 mM NaCl, 10% glycerol, 0.1% sodium N-lauroyl-sarcosinate, 1 mM PMSF and 1 mM oxidized gluthation). The lysate was applied to a 2 ml Ni-NTA column (Qiagen) equilibrated with buffer 1. The column was washed with buffer 2 (buffer 1 with 0.1% Triton-X100 instead of 0.1% sodium N-lauroyl-sarcosinate). After this, the column was washed with buffer 2, supplemented with 10 mM imidazole. Recombinant metacaspases were eluted with 300 mM imidazole in buffer 2 and checked by 12% PAGE.

For rabbit polyclonal antisera, 400 µg of purified recombinant metacaspase per rabbit was used as immunogen (Eurogentec, Herstal, Belgium).

Metacaspase-binding scFv antibodies were selected from a naive human scFv phage display library by panning. Briefly, protein antigens were coated at a concentration of 2.5-100 µg/ml in 2 ml Phosphate Buffered Saline (PBS) in immunotubes for 16-18 hours at 4°C. The tubes were washed 3 times with PBS and blocked with 4 ml 2% Skim Milk in PBS (SM-PBS). 7.5 x 10¹² phages were incubated in the immunotube, in 2 ml 2% SM-PBS for 2 hours at room temperature. The tubes were washed 10 times with 4 ml 0.1% Tween20 in PBS (T-PBS), and 5 times with 4 ml PBS. Bound phages were eluted with 1 ml 100 mM triethylamine for 5 min at room temperature, and neutralized immediately with 0.5 ml 1M Tris-HCl pH 7.4. TG1 cells were infected with the eluted phages, and a new phage stock was prepared for the next panning round. Two to three panning rounds were performed, before individual clones were tested in ELISA. Positive clones were further analyzed by *M*val fingerprinting. ScFv stocks were prepared by scFv production in E. coli HB2151 containing the pHEN2-scFv phagemid. Periplasmic extracts containing the scFv were prepared according to the Expression Module of the RPAS kit (Amersham Pharmacia Biotech).

### Agroinfiltration and expression of metacaspases in N. benthamiana and mammalian cells

The cDNAs for the metacaspases were cloned into the binary vector pB7WG2D (Karimi *et al.,* 2002). This vector carries an expression cassette for CaMV35S-driven constitutive expression of the cloned cDNA, a separate expression cassette for *EgfpER* under transcriptional control of the ro/D promoter, and the selectable marker *bar* under control of the nos promoter, the whole flanked by nopaline-type T-DNA left and right borders for efficient transfer and genomic insertion of the contained sequence.

Binary vectors were transformed into *Agrobacterium tumefasciens* strain LBA4404 supplemented with a constitutive virGN54D mutant gene (van der Fits *et al,* 2000). For infiltration, bacteria were grown until exponential growth phase, washed and diluted to an OD₆₀₀ of 0.2 in 10mM MES pH5.5, 10 mM MgSO₄, and bacterial suspensions were injected into mature leaves of 5-week-old *Nicotiana benthamiana* by applying gentle pressure on the abaxial side of the leaves using a 1 ml-syringe. Plants were kept under a 16 h light/8 h dark regime at 22°C and 70% humidity (Yang *et al.,* 2000).

For mammalian overexpression, cDNAs for metacaspases were cloned into pDEST26 (Invitrogen, Gaithersburg, MD, USA), resulting in an N-terminal HIS6-fusion under transcriptional control of the constitutive CMV promoter. Human embryonic kidney cells 293T were cultured in DMEM supplemented with 2 mM L-glutamine, 10 % fetal calf serum, 106 U/I streptomycin, 100 mg/ml penicillin and 0.4 mM sodium pyruvate. 5 x 10⁵ cells (6 well plate) were seeded and next day transfected using the calcium phosphate method as described previously (Van de Craen *et al.,* 1998).

### Immunoblot analysis

For bacterial expression analysis, induced bacterial cultures harboring the pDEST17 expression plasmid were collected after 1 to 3 hours by centrifugation and resuspended in PBS to an OD600 of 10. 5 µl per sample were loaded on gel, and after blotting analyzed with mouse penta-HIS-specific antibodies (Qiagen, Hilden, Germany).

For plant expression analysis, leaves were harvested and snap-frozen in liquid nitrogen. Extracts were prepared by grinding material and extracting with protein extraction buffer (10 mM Tris.Cl pH 7.5, 200 mM NaCl, 5 mM EDTA, 10% glycerol, 0.1% Triton-X100, 1 mM oxidized gluthation, Complete™ protease inhibitor cocktail, Roche Applied Science, Mannheim, Germany). For mammalian expression analysis, cells were scraped in medium from the plate and harvested by centrifugation. The cell pellet was lysed by adding 150 µl lysis buffer (1% NP-40, 200 mM NaCl, 10 mM Tris HCl pH 7.0, 5 mM EDTA, 10 % glycerol supplemented freshly with 1 mM PMSF, 0.1 mM aprotinin and 1 mM leupeptin). Typically 10-20 µg total protein were loaded and run on a 12% polyacrylamide gel, blotted, and metacaspases were detected using mouse penta-HIS-specific antibodies (Qiagen, Hilden, Germany), rabbit antiserum (1/2000 dilution) or cMyc-tagged monodonal single-chain antibodies (1/2000) together with mouse anti-cMyc (done 9E10, Sigma). Appropriate HRP-conjugated secondary antibodies were from Amersham Pharmacia Biotech (Roosendaal, The Netherlands). Human PARP-1 was detected with the mouse monoclonal antibody C-2-10 (Biomol Research Laboratories, Inc., PA, USA).

### Purification and N-terminal peptide sequencing of metacaspase fragments

Automated N-terminal Edman degradation of the immobilized proteins was performed on a 476A pulsed liquid sequenator equipped with an on-line phenylthiohydantoin-derivative analyser (Applied Biosystems, Foster City, CA). Prior to Edman degradation the blots were sequentially washed with water and methanol. Mass determination has been performed on a 4700 proteomics analyzer (Applied Biosystems, Foster City, CA), using the linear mode. External calibration was done with myoglobin. The p10-like fragment of Atmc9 could be purified via reversed phase HPLC in two steps as follows: Ni-NTA-purified bacterially produced protein was applied on a PLRP-S column (Polymer Labs, 4.6x200mm, eluent A= 0.1% TFA, eluent B= 90% isopropanol in 0.07% TFA) to separate the mixture in two fractions, i.e. fraction 1 between -80-85% eluent B and fraction 2 between ∼85 and 95% eluent B. The second fraction was then separated using a µRPC column (Amersham Biotech, 4.6x100mm, eluent A 0.1% TFA, eluent B 90% MeCN in 0.1% TFA). The p10-like fragment was eluted as a single peak at ∼60% eluent B.

### Metacaspase assays, substrates, and inhibitors

All tested fluorogenic substrates were from Bachem (Bubendorf,Switzerland) and inhibitors from Sigma-Aldrich (St. Louis, MO, USA), except for the caspase inhibitors, Z-FA-fmk and Z-FK-2,4,6-trimethylbenzoyloxymethyl ketone (Z-FK-tbmk) from Enzyme Systems Products (Livermore, CA, USA). Assays were performed in 150 µl with 400 ng of purified Atmc9 and 50 µM substrate in an optimized metacaspase 9 assay buffer (50 mM MES pH 5.3, 10% (w/v) sucrose, 0.1%(w/v)3-[(3-cholamidopropyl)dimethylammonio]-1-propanesulfonate [CHAPS], 10 mM DTT). Time-dependent release of free amido-4-methylcoumarin (AMC) was measured on a Cytofluor 4000 fluorescence microplate reader (PerSeptive Biosystems, Farmingham, MD, USA).

### GFP fusions, generation of transgenic BY-2 lines, and subcellular localization

The cDNAs for the studied metacaspases lacking the stop codon were cloned into the binary vector pK7FWG2 (Karimi *et a*/*.,* 2002), resulting in the C-terminal fusion of the enhanced green-fluorescent protein (*Egfp*) cDNA under control of the constitutive cauliflower mosaic virus 35S promotor. Binary vectors were transformed into *Agrobacterium tumefaciens* strain LBA4404 supplemented with a constitutive *virG* N54D mutant gene (van der Fits *et al.,* 2000). Suspension-cultured tobacco (*Nicotiana tabacum* L.) BY-2 cells were grown and transformed as described (Geelen and Inzé, 2001). Confocal laser scanning microscopy analysis was performed on a LSM510 microscopy system (Zeiss, Jena, Germany) composed of an Axiovert inverted microscope equipped with an argon ion laser as an excitation source and a 60x water immersion objective. BY-2 cells expressing GFP fusions were excited with a 488-nm laser line.

GFP emission was detected with a 505- to 530-nm band-pass filter. The images were captured with the LSM510 image acquisition software (Zeiss).

### Example 1: Identification and cloning of Arabidopsis thaliana metacaspases

Using the sequences of eight *Arabidopsis metacaspases* as reported by Uren *et al.* (2000), a sequence homology search (blastp, Altschul *et al*., 1997) was performed against an in-house collection of protein sequences corresponding to predicted *Arabidopsis* protein -encoding genes (EUGENE, Schiex *et al.,* 2001). This resulted in the detection of one extra putative metacaspase gene (genes named Atmc1 to -9). The alignment of the corresponding protein sequences is shown in Figure 1, and the corresponding family tree in Figure 2. RT-PCR was performed on pooled first-strand cDNA derived of *Arabidopsis* roots, leaves and inflorescences. Except for Atmc8, we obtained PCR products of the predicted length with all primer pairs. Several attempts to isolate cDNA for Atmc8 failed. This could mean that Atmc8 is only expressed under specific conditions, that gene prediction is not correct for Atmc8, or that it is a pseudogene. Until now, no ESTs corresponding to Atmc8 are present in public databases. Using semi-quantitative RT-PCR, attempts were made to see whether messenger RNA for the metacaspases were modulated during certain cell death-inducing conditions like H₂O₂ treatment, challenge with pathogens *(Botrytis, Alternaria, Plectosphaerella* and virulent and avirulent *Pseudomonas* strains), as well as in prolonged culturing of *Arabidopsis* cell suspension. However, we could not observe any consistent modulation of metacaspases mRNAs under these conditions. A more detailed and precise analysis will therefore be necessary to monitor subtle changes.

The nine metacaspases genes are localized on chromosomes I, IV and V. Previous genomic analysis revealed that the *Arabidopsis* genome consists of a large number of duplicated blocks, which might be the results of one or many complete genome duplications (AGI, 2000; Raes *et al*, 2003; Simillion *et al.,* 2002). Comparison of the genomic organization of all nine *Arabidopsis* metacaspases and these duplicated segments shows that Atmc8 gene is linked with genes Atmc4 to -7 by an internal duplication event on chromosome I. In addition, genes Atmc4 to -7 are organized in tandem within a region of 10.6 kb on chromosome I. Taking into account the family tree topology (see Figure 2) and this genomic organization, we conclude that this metacaspase duster (genes Atmc4-7) originated through a block duplication of the Atmc8 gene which was followed by a tandem duplication.

### Example 2: Analysis of the primary structure of metacaspases

Three of the *Arabidopsis* metacaspases (1 to 3) possess an N-terminal extension as compared to the other six proteins, and were previously termed "type I metacaspases" (Figure 2) (Uren *et al.,* 2000). These extensions could represent a prodomain, also present in mammalian upstream "initiator" caspases and as such possibly responsible for protein-protein interactions between metacaspases and oligomerizing components of different signaling complexes, resulting in subsequent metacaspase activation (Eamshaw *et al*., 1999). The *Arabidopsis* metacaspase "prodomains" contain two putative CxxC-type zinc finger structures - one of which is imperfect for Atmc3, and as such are similar to the Lsd-1 protein, a negative regulator of HR with homology to GATA-type transcription factors (Uren *et al.,* 2000; Dietrich *et al.,* 1997). Furthermore, the prodomains are rich in proline (Atmc1 and 2) or glutamine (Atmc3). The remaining metacaspases (4 to 9) lack this "prodomain" and were appointed to as "type II" metacaspases (29).

Immediately following the prodomain a conserved region of approximately 160 amino acids can be observed, corresponding to calculated molecular weights of around 17 kDa, which corresponds to the molecular weight of p20 subunits of most mammalian caspases (Earnshaw *et al.,* 1999). Carboxy-terminally, another region of homology exists, 140 residues long, with calculated molecular weights of -15 kDa, reminiscent of the p10 of caspases. In between these putative p20 and p10 domains, a region exists that differs considerately between type I and II metacaspases. While type I metacaspases have a putative linker of approximately 20 amino acids, the linker in type II metacaspases is between 90 (for Atmc9) and 150 residues long.

### Example 3: Evolutionary analysis of metacaspases

To determine the evolutionary relationship of the *Arabidopsis* metacaspases with other organisms, phylogenetic trees were constructed. As sequence data for many organisms is not complete, only the p20 region was used for alignment. Figure 3 shows an unrooted maximum-likelihood tree with metacaspases from plants, fungi, *Euglenozoa, Rhodophyta,* Alveolata and related proteases from prokaryotes. The type I metacaspases occur in a broad range of taxa (budding and fission yeast, plants, *Trypanosoma* and *Plasmodium*)*,* whereas type II metacaspases, characterized by the absence of a prodomain, are specific to plants, and can be found in monocots, dicots, mosses and ferns. Due to the incomplete sequence data in public databases, the alignment used for the generation of the phylogenetic tree in Figure 3 could not lead to the conclusion whether known metacaspases from the green alga *Chlamydomonas* and the red alga *Porphyra* were type I or type II. Nevertheless, careful analysis of the available sequences suggests that both are of type II. Additional but incomplete EST sequence data also reveal that these algae both possess at least one gene for a type I metacaspase as well.

### Example 4: Bacterial overexpression of Arabidopsis thaliana metacaspases leads to cystein-dependent autocatalytic processing

We initiated a biochemical analysis by overexpression of HIS-tagged versions of all available *Arabidopsis* metacaspases in bacteria. In parallel, mutant forms in which the presumed catalytic cysteine (Cys₂₂₀, Atmc1 numbering) is replaced by an alanine (C/A mutation) were produced. Figure 4 shows immunoblots using anti-HIS antibodies on whole bacterial lysates overproducing Atmc1, -2 and -3 (type I) and Atmc9 (type II). Overproduction of type I metacaspases results in the detection of a band at 53 kDa for Atmc1 and -3, and 58 kDa for Atmc2, corresponding to the HIS-tagged full-length proteins. At the lower region of the blot, HIS₆-positive fragments of less than 10 kDa could be detected, probably as the result of aspecific degradation by bacterial proteases. Mutation of the presumed catalytic cysteine to alanine had no effect on this pattern. For Atmc9, overproduction leads to the detection of the full-length protein (46 kDa) and a HIS-tagged fragment of 28 kDa. This fragment could result from proteolysis between the putative p20 and p10 regions. When purified recombinant HIS-tagged Atmc9 was analyzed by PAGE and silver staining, an additional fragment of approximately 16 kDa could be detected, suggesting that both p20- and p10-like fragments are generated by autoprocessing. Interestingly, for Atmc9C/A, no such processing occurs, showing that it is the result of cysteine-dependent autocatalytic action of Atmc9.

### Example 5: Overexpression of metacaspases in mammalian cells leads to cystein-dependent auto-catalytic processing, but not to cell death

Overexpression of caspases in mammalian cells often leads to auto-activation of the expressed caspase and subsequent cell death (Eamshaw *et al.,* 1999). Because of the structural homology between metacaspases and caspases, and the above observations that overexpression of metacaspases, at least for type II, results in the generation of p10 and p20 look-alikes, we tested whether plant metacaspases are active in a mammalian context. To that purpose, the N-terminally HIS₆-tagged cDNA's for wild-type and C/A mutants of Atmc1 and -9, under transcriptional control of the constitutive CMV promoter, were transfected into a human embryonic kidney cell line, 293T. Cells were followed for morphological changes typical for apoptosis in these cells, being blebbing of the plasma membrane, cytosolic condensation and the fragmentation into apoptotic bodies. However, no dear effect could be detected until up to 72 hours post-transformation. In parallel, expression levels of the metacaspases 48 hours post-transfection were analyzed by Western blotting. Polyclonal anti-Atmc1 antibodies recognized both wild-type and C/A mutant HIS-tagged Atmc1 only as full-length (52 kDa apparent MW), and no processing could be seen (Figure 5). In contrast, using a monoclonal antibody, wild-type Atmc9 (46 kDa apparent MW) could be shown to undergo proteolysis, resulting in the generation of a fragment of approximately 28 kDa. As detection with anti-HIS antibody revealed that this fragment is derived from the N-terminus of the proform (Figure 5), this means that, like with bacterial overexpression of Atmc9, probably the p10 subunit and the putative linker are removed. No p10-like fragment could be detected, most probably because the monoclonal antibody only recognizes an epitope within the p20 domain. Atmc9C/A did not show any processing, consolidating the necessity for the catalytic cysteine for autocatalytic processing of type II metacaspases.

As cell death was not evident on a morphological basis, we checked whether proteolytic cleavage of poly(ADP ribose) polymerase-1 (PARP-1) could be detected. During mammalian apoptosis, PARP-1 is cleaved by caspases 3 and 7 into fragments of 89 and 14 kDa, and this processing is often used as a hallmark for apoptotic cell death (Kaufmann *et al.,* 1993; Lazebnik *et al.,* 1994; Germain *et al*., 1999). Using a monoclonal antibody recognizing the amino-terminal part of human PARP-1, full-length PARP-1 could be detected as a band at 115 kDa in all samples. However, when Atmc9 was overexpressed, a fragment of approximately 62 could be detected. This is completely reminiscent of the necrotic cleavage of PARP-1, as reported previously (Gobeil *et al.,* 2001, Casiano *et al.,* 1998). At 48 h post-transfection, this PARP-1 cleavage was hardly detectable in any of the other samples. At 72 h post-transfection, PARP-1 cleavage could also be detected in other samples, albeit to a lesser extent (not shown). Probably, this necrotic processing is the consequence of cell death by nutrient starvation, which is accelerated by active Atmc9. We conclude that, either directly or indirectly, overexpression in human cells of an active form of Atmc9 leads to necrotic processing of PARP-1.

Lysates from 293T cells overexpressing metacaspases were also incubated with different synthetic fluorigenic substrates for caspases, namely acetyl-Asp-Glu-Val-Asp-aminomethylcoumarin (Ac-DEVD-amc), acetyl-IIe-Glu-Thr-Asp-aminomethylcoumarin (Ac-IETD-amc), Acetyl-Leu-Glys-His-Asp-aminomethylcoumarin (Ac-LEHD-amc), acetyl-Trp-Glu-His-Asp-aminomethylcoumarin (Ac-WEHD-amc), Acetyl-Tyr-Val-Ala-Asp-aminomethylcoumarin (Ac-YVAD-amc) and benzyloxycarbonyl-Val-Ala-Asp-aminomethylcoumarin (zVAD-amc). However, no significant increase in activity could be measured with any of these substrates, while lysates from cells overexpressing murine caspase-3 showed dear DEVD-ase activity. Therefore, although *Arabidopsis thaliana* metacaspase 9 (Atmc9) overexpression results in autoprocessing, this does not lead to classical caspase-like activity.

### Example 6: Overexpression of metacaspases in Nicotiana benthamiana leads to cysteine-dependent processing, but not to cell death

Next, we introduced the ORFs for Atmc1 and -9, under transcriptional control of the constitutive 35S CaMV promoter, into tobacco leaves by *Agrobacterium* infiltration. To investigate the role of the presumed catalytic cysteine, Atmc1C/A and -9C/A mutants were also tested.

Plants were visually scored for the appearance of necrotic lesions during the following days.

However, no consistent effect of overexpression of metacaspases could be observed.

Therefore, expression levels of the different proteins were assessed by Western analysis.

Using parallel overexpression of GFP, transformation efficiency was shown to be similar in all setups. Expression of the different Atmc's was analyzed using metacaspase-specific antisera.

As shown in Figure 6, both wild-type Atmc1 and Atmc1C/A were present as full-length precursor (39 kDa), and no p20- and p10-like fragments could be detected. Both forms showed partial degradation, probably due to aspecific degradation. Therefore we conclude that, as in bacteria and mammalian cells, overexpression of type I metacaspases in insufficient for autocatalytic processing. However, in the case of Atmc9, full-length protein (36 kDa) could only be seen for the C/A mutant. Upon overexpression, wild-type Atmc9 is processed into fragments of approximately 22 and 14 kDa. The fragment of 22 kDa could represent the N-terminal half of Atmc9, corresponding to the HIS-tagged 28 kDa band in bacterial and mammalian lysate, and thus be the plant counterpart of the p20 of activated mammalian caspases. However, comparing to bacterial and mammalian overexpression, there seems to be a small discrepancy in the size of the putative p10-like fragment. As mentioned above, besides an N-terminal 28 kDa fragment, bacterial production of processed Atmc9 also yielded a peptide of 16 kDa, while in plants, a 14 kDa-fragment could be detected. This could mean that in contrast to bacteria, additional processing occurs in plants, resulting in a lower molecular weight. These results show that overexpression of type II Atmc's in plants, like in bacteria and mammalian cells, leads to cysteine-dependent auto-processing.

Despite repeated overexpression experiments using different titers of *Agrobacterium* and plant growth conditions, and the fact that autocatalytic processing was triggered upon metacaspase overexpression, no concomitant cell death could be seen. We therefore conclude that mere over-expression of type II metacaspases may be sufficient for autocatalytic processing, but that this does not result in cell death.

### Example 7: Autocatalytic processing of Atmc9 occurs after arginine and lysine

As bacterial overproduction of Atmc9 is sufficient for autoprocessing, we characterized the putative p20 and p10 fragments by N-terminal peptide sequencing and by determination of their molecular mass by mass-spectrometry. When HIS-tag-purified Atmc9 was analyzed on PAGE and silver staining, major fragments with apparent molecular masses of 28, 21 and 16 kDa were visible (nor shown). Previous experiments revealed that of these, only the 28 kDa fragment could be detected with anti-HIS₆ antibody, and thus could represent the HIS-tagged p20-like subunit. Therefore we reasoned that the band at 21 kDa could represent the mature p20, i.e. after removal of the HIS-tag and a short prodomain. The other fragment, at 16 kDa, could then be the p10-like subunit. The p10-like fragment could be purified sufficiently to directly submit it to Edman degradation sequencing. This resulted in the peptide sequence ALPFKAV, which indicates that the p10 is generated by cleavage after Arg₁₈₃. As can be seen on the sequence alignment in Figure 1, all type II metacaspases possess either an arginine or a lysine at this position, strongly suggesting that metacaspases are arginine/lysine-specific proteases. Molecular mass determination by MALDI-TOF/TOF revealed the mass of the p10-like subunit of Atmc9 to be 15442 Da, which demonstrates that it indeed consists of amino acids 184 to 325 (calculated mass 15427 Da).

As the putative p20 subunit could not be purified, separation by PAGE was necessary first to isolate this fragment. Peptide sequencing revealed that the N-terminus of this fragment was generated by cleavage in the short linker between the HIS₆-tag and Atmc9, more precisely after the two lysines in the sequence MSYYHHHHHHLESTSLYKKAGSTM, where the last methionine is the start of Atmc9. However, as can be seen on Figure 1, a similar peptide sequence K[K/R][A/L] can be found four residues further downstream in most type II metacaspases, and in the aligned type I metacaspases. This suggests that, as a result of the addition of the HIS₆-tag, a novel and probably more accessible cleavage site was created which is similar to the natural site.

The autocatalytic cleavage sites of Atmc9 are shown on Figure 1. These results indicate that, although structural homology exists between mammalian caspases and metacaspases, their substrate specificity is different, with an absolute necessity for Lys or Arg in the P1 position.

### Example 8: Cleavage of artificial substrates by Atmc9

Several artificial substrates of Atcm9 were tested and the *kcat*/*Km* was determined.

The activity assay buffer used was composed of 50 mM MES pH 5.3, 10% (w/v) sucrose, 0.1% (w/v) 3-[(3-cholamidopropyl)dimethylammonio]-1-propanesulfonate [CHAPS], 10 mM DTT.

The concentration of active sites in the preparation of rAtmc9 was determined by active site titration with the irreversible inhibitor Z-FK-2,4,6-trimethylbenzoyloxymethyl ketone (Z-FK-tbmk; Enzyme Systems Products, Livermore, CA, USA) to be 30 µM.

For determination of *kcatl Km,* 50 µl of 20 µM of the tested substrates (t-butyloxycarbonyl-Gly-Lys-Arg-7-amido-4-methylcoumarin (Boc-GKR-AMC); t-butyloxycarbonyl-Gly-Arg-Arg-7-amido-4-methylcoumarin (Boc-GRR-AMC); benzyloxycarbonyl-Phe-Arg-AMC (Z-FR-AMC); H-Ala-Phe-Lys-7-amido-4-methylcoumarin (H-AFK-AMC)) was mixed with 50 µl 600 nM rAtmc9 (final concentrations 300 nM rAtmc9 and 10 µM substrate). Release of free AMC was determined in a time course using a FLUOstar Optima fluorescence plate reader (BMB Labtechnologies, Offenburg, Germany). After total hydrolysis of the substrates, *kcat*/*Km* was calculated using the following formula: *kcat*/*Km*=*kobs*/Et, where *kobs* is determined as the decrease per second of the natural logarithm of substrate left, and Et is the concentration of enzyme active sites in the reaction.

Table 1 shows that both Boc-GRR-AMC and Z-FR-AMC are good substrates for Atmc9, while Boc-GKR-AMC is a somewhat less good substrate. Although H-AFK-AMC is less efficiently cleaved by Atmc9, these results directly demonstrate that Atmc9 is an arginine- and lysine-specific protease.

**Table 1: kcat/Km of artificial substrates of Atmc9**

| Substrate | *kcat*/*Km* (mM⁻¹.s⁻¹) |
|---|---|
| Boc-GRR-AMC | 2.90 |
| Boc-GKR-AMC | 1.90 |
| Z-FR-AMC | 2.90 |
| H-AFK-AMC | 0.75 |

### Example 9: Atmc9 has an acidic pH optimum

To further characterize Atmc9 biochemically, the purified protein and its cysteine mutant were tested for their ability to cleave the synthetic fluorogenic oligopeptide substrate t-butyloxycarbonyl-GKR-7-amido-4-methylcoumarin (Boc-GKR-AMC) at different pH. As shown in Figure 7, Atmc9 clearly has GKR-ase activity while the catalytic cysteine mutant Atmc9C/A does not. Interestingly, the pH optimum for Atmc9 activity is 5.3, whereas activity at the physiological pH of the cytoplasm (7.0-7.5) is completely abolished.

Activation by acidic pH has also been observed for human caspase 3 (Roy *et al.,* 2001). In this case, a so-called "safety catch" hinders both the autocatalytic maturation as well as the vulnerability to proteolytic activation by upstream proteases. However, while this activation is stable in the case of caspase 3, i.e. once mature the protease shows optimal activity at pH 7.0-8.0 (Garcia-Calvo *et al.,* 1999), preincubation of Atmc9 at low pH is not sufficient to irreversibly activate it.

### Example 10: Subcellular localization of metacespases

Because Atmc9 is only active at low pH, it was checked if it was localized in the central vacuole. Therefore, C-terminal green fluorescent protein (GFP) fusions of Atmc9, and in parallel Atmc1, Atmc2 and Atmc3, were overproduced in tobacco Bright Yellow 2 (BY-2) cells and their subcellular localization determined by confocal laser scanning microscopy (Figure 8).

In the case of Atmc9, high fluorescence could be seen in the nucleus, although a significant fraction of the protein seemed to be present in the cytoplasm. The subcellular localization pattern of the inactive C/A mutant of Atmc9 was identical to that of the wild-type protein, thereby excluding leakage of free GFP or a p15-GFP fusion protein from the nucleus to the cytoplasm as a consequence of autoprocessing. More important, no fluorescence was detected in the central vacuole. For Atmc1, the protein was mostly localized In the nucleus, with only minor fluorescence in the cytoplasm. In contrast, both Atmc2 and Atmc3 were largely excluded from the nucleus and remained in the cytoplasm. These data were confirmed by subcellular fractionation of wild-type *Arabidopsis* plants and Western blotting.

### References

AGI. (2000) Analysis of the genome sequence of the flowering plant Arabidopsis thaliana. Nature 408, 796-815.
Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. Nucleic Acids Res. 25, 3389-3402.
Buckner, B., Johal, G.S. and Janick-Buckner, D. (2000) Cell death in maize. Physiol. Plant. 108, 231-239.
Casiano, C. A., Ochs, R. L., and Tan, E. M. (1998) Distinct cleavage products of nuclear proteins in apoptosis and necrosis revealed by autoantibody probes. Cell Death Differ. 5, 183-190
Cohen, G.M. (1997) Caspases: the executioners of apoptosis. Biochem. J. 326, 1-6.
Danon, A. and Gallois, P. (1998) UV-C radiation induces apoptotio-like changes in Arabidopsis thaliana. FEBS Lett. 437, 131-136.
De Jong, A.J., Hoeberichts, F.A., Yakimova, E.T., Maximova, E. and Woltering, E.J. (2000) Chemical-induced apoptotic cell death in tomato cells: involvement of caspase-like proteases. Planta, 211, 656-662.
del Pozo, O. and Lam, E (1998) Caspases and programmed cell death in the hypersensitive response of plants to pathogens. Curr. Biol. 8, 1129-1132.
Dietrich, R.A., Richberg, M.H., Schmidt, R., Dean, C. and Dangl, J.L. (1997) A novel zinc finger protein is encoded by the Arabidopsis LSD1 gene and functions as a negative regulator of plant cell death. Cell, 88, 685-694.
Eamshaw, W.C., Martins, L.M. and Kauffmann, S.H. (1999) Mammalian caspases: structure, activation, substrates, and functions during apoptosis. Annu. Rev. Biochem. 68, 383-424.
Eddy, S. R. (1998) Profile hidden Markov models. Bioinformatics 14, 755-763
Ellis, R. E., Yuan, J. Y., and Horvitz, H. R. (1991) Mechanisms and functions of cell death. Annual Review of Cell Biology 7, 663-698
Fath, A., Bethke, P., Lonsdale, J., Meza-Romero, R., and Jones, R. (2000) Programmed cell death in cereal aleurone. Plant Mol. Biol. 44, 255-266
Fiers, W., Beyaert, R., Declercq, W. and Vandenabeele, P. (1999) More than one way to die: apoptosis, necrosis and reactive oxygen species. Oncogene, 18, 7719-7730.
Fukuda, H. (2000) Programmed cell death of tracheary elements as a paradigm in plants. Plant Mol. Biol. 44, 245-253.
Garcia-Calvo, M., Peterson, E.P., Rasper, D.M., Vaillancourt, J.P., Zamboni, R., Nicholson, D.W. and Thomberry, N.A. (1999) Purification and catalytic properties of human caspase family members. Cell Death Differ. 6, 362-369.
Geelen, D.N.V. and Inzé, D.G. (2001) A bright future for the Bright Yellow-2 cell culture. Plant Physiol. 127, 1375-1379.
Germain, M., Affar, E. B., D'Amours, D., Dixit, V. M., Salvesen, G. S., and Poirier, G. G. (1999) Cleavage of automodified poly(ADP-ribose) polymerase during apoptosis. Evidence for involvement of caspase-7. J. Biol. Chem. 274, 28379-28384
Gobeil, S., Boucher, C. C., Nadeau, D., and Poirier, G. G. (2001) Characterization of the necrotic cleavage of poly(ADP-ribose) polymerase (PARP-1): implication of lysosomal proteases. Cell Death Differ. 8, 588-594
Greenberg, J. T. (1996) Programmed cell death: a way of life for plants. Proc. Natl. Acad. Sci. USA 93, 12094-12097
Greenberg, J. T. (1997) Programmed cell death in plant-pathogen interactions. Annual Review of Plant Physiology and Plant Molecular Biology 48, 525-545
Hall, T.A. (1999) BioEdit: a user-friendly biological sequence alignment editor and analysis program for Windows 95/98/NT. Nucleic Acids Symp. Ser. 41, 95-98.
Heath, M. C. (2000) Hypersensitive response-related death. Plant Mol. Biol. 44, 321-334
Jabs, T. (1999) Reactive oxygen intermediates as mediators of programmed cell death in plants and animals. Biochem. Pharmacol. 57, 231-245.
Karimi, M., Inzé, D. and Depicker, A. (2002) GATEWAY™ vectors for Agrobacterium-mediated plant transformation. Trends Plant Sci. 7,193-195.
Katsuhara, M. (1997) Apoptosis-like cell death in barley roots under salt stress. Plant Cell Physiol. 39, 1091-1093.
Kaufmann, S. H., Desnoyers, S., Ottaviano, Y., Davidson, N. E., and Poirier, G. G. (1993) Specific proteolytic cleavage of poly(ADP-ribose) polymerase: an early marker of chemotherapy-induced apoptosis. Cancer Res. 53, 3976-3985
Korthout, H.A.A.J., Berecki, G., Bruin, W., van Duijn, B. and Wang, M. (2000) The presence and subcellular localization of caspase 3-like proteinases in plant cells. FEBS Lett. 475, 139-144.
Kratsch, H.A. and Wise, R.R. (2000) The ultrastructure of chilling stress. Plant Cell Environ, 23, 337-350.
Lazebnik, Y. A., Kaufmann, S. H., Desnoyers, S., Poirier, G. G., and Eamshaw, W. C. (1994) Cleavage of poly(ADP-ribose) polymerase by a proteinase with properties like ICE. Nature 371, 346-347
Lockshin, R.A. and Zakeri, Z. (2002) Caspase-independent cell deaths. Curr. Opin. Cell Biol. 14, 727-733.
Lukashin, A. V., and Borodovsky, M. (1998) GeneMark.hmm: new solutions for gene finding. Nucleic Acids Res. 26, 1107-1115.
Morel, J.-B., and Dangl, J. L. (1997) The hypersensitive response and the induction of cell death in plants. Cell Death Differ. 4, 671-683
O'Brien, I.E.W., Murray, B.G., Baguley, B.C., Morris, B.A.M. and Ferguson, I.B. (1998) Major changes in chromatin condensation suggest the presence of an apoptotic pathway in plant cells. Exp. Cell Res. 241, 46-54.
Pellinen, R., Palva, T. and Kangasjärvi, J. (1999) Subcellular localization of ozone-induced hydrogen peroxide production in birch (Betula pendula) leaf cells. Plant J. 20, 349-356.
Pennell, R.I. and Lamb, C. (1997) Programmed cell death in plants. Plant Cell, 9, 1157-1168. **Raes, J., and Van de Peer, Y.** (1999) *http:*//*www.ebi.ac.uk*/*embnet.news*/*vol6 1*/*ForConlbody forcon.html.*
Raes, J., Vandepoele, K., Simillion, C., Saeys, Y. and Van de Peer, Y. (2003) Investigating ancient duplication events in the Arabidopsis genome. J. Struct. Funct Genomics 3, 117-129.
Rogl, H., Kosemund, K., Kühlbrandt, W. and Collinson, I. (1998) Refolding of Escherichia coli produced membrane protein inclusion bodies immobilised by nickel chelating chromatography. FEBS Lett. 432, 21-26.
Roy, S., Bayly, C.I., Gareau, Y., Houtzager, V.M., Kargman, S., Keen,S.L.C., Rowland, K., Seiden, I.M., Thomberry, N.A. and Nicholson,D.W. (2001) Maintenance of caspase-3 proenzyme dormancy by an intrinsic "safety catch" regulatory tripeptide. Proc. Natl. Acad. Sci. USA, 98, 6132-6137.
Schiex, T., Moisan, A. and Rouzé, P. (2001) EUGÈNE: an eukaryotic gene finder that combines several sources of evidence. Lect. Notes Comput. Sci. 2066, 111-125.
Schmidt, H. A., Strimmer, K., Vingron, M., and von Haeseler, A. (2002) TREE-PUZZLE: maximum likelihood phylogenetic analysis using quartets and parallel computing. Bioinformatics 18, 502-504
Simillion, C., Vandepoele, K., Van Montagu, M. C., Zabeau, M., and Van De Peer, Y. (2002) The hidden duplication past of Arabidopsis thaliana. Proc. Natl. Acad. Sci. USA 99, 13627-13632
Solomon, M., Belenghi, B., Delledonne, M., Menachem, E. and Levine, A. (1999) The involvement of cysteine proteases and protease inhibitor genes in the regulation of programmed cell death in plants. Plant Cell, 11, 431-443.
Thompson, J. D., Higgins, D. G., and Gibson, T. J. (1994) CLUSTAL W: improving the sensitivity of progressive multiple sequence alignment through sequence weighting, position-specific gap penalties and weight matrix choice. Nucleic Acids Res. 22, 4673-4680.
Thompson, J.D., Gibson, T.J., Plewniak, F., Jeanmougin, F. and Higgins, D.G. (1997) The CLUSTAL_X windows interface: flexible strategies formultiple sequence alignment aided by quality analysis tools. Nucleic Acids Res. 25, 4876-4882.
Uren, A.G., O'Rourke, K., Aravind, L., Pisabarro, M.T., Seshagiri, S., Koonin, E.V. and Dixit, V.M. (2000) Identfication of paracaspases and metacaspases: two ancient families of caspase-like proteins, one of which plays a key role in MALT lymphoma. Mol. Cell, 6, 961-967.
Utz, P.J. and Anderson, P. (2000) Life and death decisions: regulation ofapoptosis by proteolysis of signaling molecules. Cell Death Differ. 7, 589-602.
Van de Craen, M., Van Loo, G., Pype, S., Van Criekinge, W., Van den brande, I., Molemans, F., Fiers, W., Declercq, W., and Vandenabeele, P. (1998) Identification of a new caspase homologue: caspase-14. Cell Death Differ. 5, 838-846
van der Fits, L., Deakin, E.A., Hoge, J.H.C. and Memelink, J. (2000) The ternary transformation system: constitutive virG on a compatible plasmid dramatically increases Agrobacterium-mediated plant transformation. Plant Mol. Biol. 43, 495-502.
Van de Peer, Y. and De Wachter, R. (1994) TREECON for Windows: a software package for the construction and drawing of evolutionary trees for the Microsoft Windows environment. Comput. Appl. Biosci. 10, 569-570.
Williams, G. T. (1994) Programmed cell death: a fundamental protective response to pathogens. Trends Microbio/. 2, 463-464
Wu, T.D. and Brutlag, D.L. (1995) Identification of protein motifs using conserved amino acid properties and partitioning techniques. In Proceedings of the Third International Conference on Intelligent Systems for Molecular Biology (ISMB-95) (Rawlings, C., Clark, D., Altman, R., Hunter, L., Lengauer, T. and Wodak, S., eds). Menlo Park: AAAI Press, pp. 402-410.
Wu, H. M., and Cheun, A. Y. (2000) Programmed cell death in plant reproduction. Plant Mol. Biol. 44, 267-281
Yang, Y., Li, R., and Qi, M. (2000) In vivo analysis of plant promoters and transcription factors by agroinfiltration of tobacco leaves. Plant J. 22, 543-551
Young, T. E., and Gallie, D. R. (2000) Programmed cell death during endosperm development. Plant Mol. Biol. 44, 283-301
Yu, X.-H., Perdue, T. D., Heimer, Y. M., and Jones, A. M. (2002) Mitochondrial involvement in tracheary element programmed cell death. Cell Death Differ. 9, 189-198

## Claims

1. The use of a metacaspase comprising SEQ ID N° 2 to process a protein at a cleavage site comprising arginine or lysine at position P1.

2. The use of a metacaspase according to claim 1, whereby said metacaspase is consisting of SEQ ID N° 1.

3. The use of a metacaspase according to any of the claims 1 or 2, whereby said metacaspase is active at an acidic pH.

4. The use of a metacaspase according to any of the claims 1, 2 or 3 to modulate cell growth.

5. The use of a metacaspase according to any of the claims 1, 2 or 3 to modulate cell death.

## Patentansprüche

1. Verwendung einer Metacaspase, die SEQ ID Nr. 2 umfasst, um ein Protein an einer Spaltstelle zu verarbeiten, die Arginin oder Lysin an Position P1 umfasst.

2. Verwendung einer Metacaspase nach Anspruch 1, wobei die Metacaspase aus SEQ ID Nr. 1 besteht.

3. Verwendung einer Metacaspase nach einem der Ansprüche 1 oder 2, wobei die Metacaspase bei einem sauren pH-Wert aktiv ist.

4. Verwendung einer Metacaspase nach einem der Ansprüche 1, 2 oder 3 zur Modulation des Zellwachstums.

5. Verwendung einer Metacaspase nach einem der Ansprüche 1, 2 oder 3 zur Modulation des Zelltods.

## Revendications

1. Utilisation d'une métacaspase comprenant la SEQ ID N° 2 pour transformer une protéine au niveau d'un site de clivage comprenant de l'arginine ou de la lysine en position P1.

2. Utilisation d'une métacaspase selon la revendication 1, par laquelle ladite métacaspase est constituée de la SEQ ID N° 1.

3. Utilisation d'une métacaspase selon l'une quelconque des revendications 1 ou 2, par laquelle ladite métacaspase est active à un pH acide.

4. Utilisation d'une métacaspase selon l'une quelconque des revendications 1, 2 ou 3 pour moduler la croissance cellulaire.

5. Utilisation d'une métacaspase selon l'une quelconque des revendications 1, 2 ou 3 pour moduler la mort cellulaire.
